(19) Europäisches Patentamt / European Patent Office / Office européen des brevets

(11) **EP 4 060 675 B1**

(12) **EUROPEAN PATENT SPECIFICATION**

(45) Date of publication and mention
of the grant of the patent:
**13.12.2023 Bulletin 2023/50**

(21) Application number: **21199728.3**

(22) Date of filing: **29.09.2021**

(51) International Patent Classification (IPC):
$A61B\ 5/021^{(2006.01)}$    $A61B\ 5/024^{(2006.01)}$
$A61B\ 5/11^{(2006.01)}$    $A61B\ 5/0245^{(2006.01)}$
$G16H\ 50/50^{(2018.01)}$    $G16H\ 40/63^{(2018.01)}$
$G16H\ 50/20^{(2018.01)}$    $A61B\ 5/00^{(2006.01)}$

(52) Cooperative Patent Classification (CPC):
**G16H 50/50; A61B 5/021; A61B 5/024;
A61B 5/1118; G16H 40/63; G16H 50/20;**
A61B 5/02416; A61B 5/02438; A61B 5/0245;
A61B 5/4035; A61B 5/7207; A61B 2505/09

(54) **ESTIMATING CARDIAC PARAMETERS WHEN PERFORMING AN ACTIVITY USING A PERSONALIZED CARDIOVASCULAR HEMODYNAMIC MODEL**

SCHÄTZUNG VON HERZPARAMETERN BEI DER DURCHFÜHRUNG EINER AKTIVITÄT UNTER VERWENDUNG EINES PERSONALISIERTEN KARDIOVASKULÄREN HÄMODYNAMISCHEN MODELLS

ESTIMATION DE PARAMÈTRES CARDIAQUES LORS DE LA MISE EN ŒUVRE D'UNE ACTIVITÉ À L'AIDE D'UN MODÈLE D'HÉMODYNAMIQUE CARDIOVASCULAIRE PERSONNALISÉ

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **15.03.2021 IN 202121010972**

(43) Date of publication of application:
**21.09.2022 Bulletin 2022/38**

(73) Proprietor: **Tata Consultancy Services Limited**
**400021 Mumbai, Maharashtra (IN)**

(72) Inventors:
• **ROY, DIBYENDU**
**700160 Kolkata, West Bengal (IN)**
• **MAZUMDER, OISHEE**
**700160 Kolkata, West Bengal (IN)**
• **SINHA, ANIRUDDHA**
**700160 Kolkata, West Bengal (IN)**
• **KHANDELWAL, SUNDEEP**
**201309 Noida, Uttar Pradesh (IN)**
• **GHOSE, AVIK**
**700160 Kolkata, West Bengal (IN)**

(74) Representative: **Goddar, Heinz J.**
**Boehmert & Boehmert**
**Anwaltspartnerschaft mbB**
**Pettenkoferstrasse 22**
**80336 München (DE)**

(56) References cited:
• **ROY DIBYENDU ET AL: "Multimodal cardiovascular model for hemodynamic analysis: Simulation study on mitral valve disorders", PLOS ONE, vol. 16, no. 3, 4 March 2021 (2021-03-04), pages 1-28, XP055905181, DOI: 10.1371/journal.pone.0247921 Retrieved from the Internet: URL:https://doi.org/10.1371/journal.pone.0 247921> [retrieved on 2022-03-24]**
• **ROY DIBYENDU ET AL: "Hemodynamic Evaluation of a Novel Neuromodulation based Adaptive controller for Mitral Stenosis", 2020 EUROPEAN CONTROL CONFERENCE (ECC), EUCA, 12 May 2020 (2020-05-12), pages 264-270, XP033795789, [retrieved on 2020-07-17]**

**Description**

CROSS-REFERENCE TO RELATED APPLICATIONS AND PRIORITY

The present application claims priority to Indian application no. 202121010972, filed on 15th March, 2021.

TECHNICAL FIELD

The disclosure herein generally relates to the field of computer assisted modeling of hemodynamic patterns of physiologic blood flow, and, more particularly, to systems and methods for estimating cardiac parameters when performing an activity using a personalized cardiovascular hemodynamic model.

BACKGROUND

Cardiovascular disease (CVD) is one of the main causes of deaths in the world today. With advancing age, the risk of CVD increases significantly. To reduce the effect of CVDs, it has been a well-established fact that the higher levels of regular physical exercises would reduce cardiovascular events and mortality. According to medical literature and guidelines, increasing levels of physical activity are the first-line approaches for preventing and treating vascular dysfunction and cardio-metabolic diseases. Moreover, during post-operative recovery of a patient who had suffered a cardiovascular surgery recently, performing prescribed exercises during the recovery phase is always recommended. However, unless the patient diligently and periodically consults a caregiver, it is challenging to gauge whether the prescribed exercises are yielding a desired outcome, or any change is required in the planned therapy. ROY DIBYENDU ET AL: "Multimodal cardiovascular model for hemodynamic analysis: Simulation study on mitral valve disorders",PLOS ONE, vol. 16, no. 3, 4 March 2021 disclose a model for an in-silico cardiac computational model to analyze the effect and severity of valvular disease on general hemodynamic parameters. They propose a multimodal and multiscale cardiovascular model to simulate and understand the progression of valvular disease associated with the mitral valve. I

ROY DIBYENDU ET AL: "Hemodynamic Evaluation of a Novel Neuromodulation based Adaptive controller for Mitral Stenosis",2020 EUROPEAN CONTROL CONFERENCE (ECC), EUCA, 12 May 2020 present a simulation platform to study and do predictive analysis on valvular heart disease, Mitral stenosis (MS) in particular and propose a control approach to correct hemodynamic imbalances during severe MS conditions. Their developed cardiovascular hemodynamics model helps to create 'what if' conditions to study the variation in different hemodynamic parameters like blood flow, aortic and ventricular pressure, etc. during normal and pathological conditions.

SUMMARY

Embodiments of the present disclosure present technological improvements as solutions to one or more of the above-mentioned technical problems recognized by the inventors in conventional systems.

In an aspect, there is provided a processor implemented method comprising the steps of: estimating, via one or more hardware processors, a plurality of input parameters for a personalized cardiovascular hemodynamic model associated with a subject being monitored, the plurality of input parameters comprising (i) Systemic Vascular Resistance (SVR), (ii) unstressed blood volume, (iii) total blood volume in a body of the subject, and (iv) heart rate of the subject, the step of estimating comprises: estimating the SVR using Metabolic EquivalenT (MET) levels associated with an activity level of the subject; and updating the unstressed blood volume estimated when the subject is at rest, using an autoregulation method, when an activity is performed by the subject, wherein the autoregulation method comprises: sensing aortic pressure by baroreceptors located at carotid sinus and aortic arch; converting the sensed aortic pressure into a neural firing frequency via afferent sympathetic pathways; generating sympathetic and parasympathetic nervous activities via a central nervous system and efferent pathway depending on the neural firing frequency; and updating an additional blood demand representing the unstressed blood volume during the activity using the generated sympathetic and parasympathetic nervous activities; and estimating, via the personalized cardiovascular hemodynamic model, cardiac parameters including cardiac output, ejection fraction and mean arterial pressure, using the estimated plurality of input parameters.

In another aspect, there is provided a system comprising: one or more data storage devices operatively coupled to one or more hardware processors and configured to store instructions configured for execution via the one or more hardware processors to: estimate a plurality of input parameters for a personalized cardiovascular hemodynamic model associated with a subject being monitored, the plurality of input parameters comprising (i) Systemic Vascular Resistance (SVR), (ii) unstressed blood volume, (iii) total blood volume in a body of the subject, and (iv) heart rate of the subject, wherein estimating the plurality of input parameters comprises: estimating the SVR using Metabolic EquivalenT (MET) levels associated with an activity level of the subject; and updating the unstressed blood volume estimated when the subject is at rest, using an autoregulation method, when an activity is performed by the subject, wherein the autoregulation method comprises: sensing aortic pressure by baroreceptors located at carotid sinus and aortic arch; converting the sensed aortic pressure into a neural firing frequency via afferent sympathetic pathways; generating sympathetic and parasympathetic nervous activities via a central nervous system and efferent pathway depending on the neural firing frequency; and updating an additional blood demand representing the unstressed blood volume during the activity using the generated sympathetic and parasympathetic nervous activities; and estimate cardiac parameters including cardiac output, ejection fraction and mean arterial pressure, using the estimated plurality of input parameters.

In yet another aspect, there is provided a computer program product comprising a non-transitory computer readable medium having a computer readable program embodied therein, wherein the computer readable program, when executed on a computing device, causes the computing device to: estimate a plurality of input parameters for a personalized cardiovascular hemodynamic model associated with a subject being monitored, the plurality of input parameters comprising (i) Systemic Vascular Resistance (SVR), (ii) unstressed blood volume, (iii) total blood volume in a body of the subject, and (iv) heart rate of the subject, wherein estimating the plurality of input parameters comprises: estimating the SVR using Metabolic EquivalenT (MET) levels associated with an activity level of the subject; and updating the unstressed blood volume estimated when the subject is at rest, using an autoregulation method, when an activity is performed by the subject, wherein the autoregulation method comprises: sensing aortic pressure by baroreceptors located at carotid sinus and aortic arch; converting the sensed aortic pressure into a neural firing frequency via afferent sympathetic pathways; generating sympathetic and parasympathetic nervous activities via a central nervous system and efferent pathway depending on the neural firing frequency; and updating an additional blood demand representing the unstressed blood volume during the activity using the generated sympathetic and parasympathetic nervous activities; and estimate cardiac parameters including cardiac output, ejection fraction and mean arterial pressure, using the estimated plurality of input parameters.

In accordance with an embodiment of the present disclosure, the one or more hardware processors are configured to personalize a cardiovascular hemodynamic model to obtain the personalized cardiovascular hemodynamic model, prior to estimating the plurality of input parameters, based on one or more of (i) cardiac parameters obtained from an echocardiogram, (ii) ECG signal obtained from a wearable device worn by the subject when performing the activity and (iii) metadata of the subject including height and weight associated thereof.

In accordance with an embodiment of the present disclosure, the one or more hardware processors are configured to perform sequential activation of a right atrium $ra$, a left atrium $la$, a right ventricle $rv$ and a left ventricle $lv$ of the personalized cardiovascular hemodynamic model using compliance functions $C_{ra}(t)$, $C_{la}(t)$, $C_{rv}(t)$ and $C_{lv}(t)$ respectively, the compliance functions being defined as:

(i) the compliance function to actuate $ra$, $C_{ra}(t) = C_{min,ra} + 0.5 \times (C_{max,ra} - C_{min,ra})u(t)$, wherein

$$u(t) = \begin{cases} 0, & 0 \leq t < T_a \\ 1 - cos\left(2\pi \frac{t-T_a}{T-T_a}\right), & T_a \leq t < T, \end{cases} \quad C_{min,ra}$$

and $C_{max,ra}$ are the minimum and maximum values of the $ra$ compliance, u(t) is the activation function, time t is considered over a complete cardiac cycle, $T_a$ is the start of the activation of $ra$ and $T$ is the end of the cardiac cycle;

(ii) the compliance function to actuate $la$, $C_{la}(t) = C_{min,la} + 0.5 \times (C_{max,la} - C_{min,la})u(t - d_{la})$, wherein $C_{min,la}$ and $C_{max,la}$ are the minimum and maximum values of the $la$ compliance and $d_{la}$ represents a time delay between activation of the $ra$ and the $la$; and

(iii) the compliance functions to actuate $rv$ and $lv$ are represented as

$$C_i(t) = C_i \times u_v(t - d), i \in \{lv, rv\},$$

$$u_v(t) = \begin{cases} 0.5 - 0.5cos\left(\pi \frac{1}{T_1}\right), & 0 \leq t < T_1 \\ 0.5 + 0.5cos\left(\pi \frac{t-T_1}{T_2-T_1}\right), & T_1 \leq t < T_2 \\ 0, & T_2 \leq t < T \end{cases}$$

wherein $C_i$; $i \in \{lv, rv\}$ is the systolic compliance across $lv$ or $rv$ and is estimated as a ratio of R-peak and T-peak of the ECG signal, $u_v(t)$ is the activation function, $d$ represents the time delay in activation of $lv$ or $rv$ from $ra$, and $T_1$ and $T_2$ are the systolic and diastolic activation time instances of the cardiac cycle respectively.

In accordance with an embodiment of the present disclosure, the one or more hardware processors are configured to estimate the total blood volume using height and weight of the subject.

In accordance with an embodiment of the present disclosure, the one or more hardware processors are con-

$$R_s(t) = \begin{cases} R_s(0) & if\ MET(t) < 2.5 \\ \frac{R_s(0)}{MET(t)} * e^{-\frac{t*MET(t)}{\tau}} & otherwise \end{cases}$$

figured to estimate the SVR based on , *otherwise*, wherein $R_s(0)$ represents the SVR at rest, *MET(t)* represents a metabolic equivalent of the activity performed at the tth time, and $\tau$ is a time constant, and wherein the SVR corresponds to a section of the body of the subject depending on the activity being performed, while the SVR of remaining sections are considered constant or modulated by the autoregulation method, the section of the body being an upper body, a middle body or a lower body of the subject.

In accordance with an embodiment of the present disclosure, the one or more hardware processors are configured to estimate the heart rate of the subject using (i) a Photoplethysmogram (PPG) signal and an accelerometer signal or (ii) an Electrocardiogram (ECG) signal, from a wearable device worn by the subject when performing the activity.

It is to be understood that both the foregoing general description and the following detailed description are exemplary and explanatory only and are not restrictive of the invention, as claimed.

BRIEF DESCRIPTION OF THE DRAWINGS

The accompanying drawings, which are incorporated in and constitute a part of this disclosure, illustrate exemplary embodiments and, together with the description, serve to explain the disclosed principles:

FIG. 1 illustrates an exemplary block diagram of a system for estimating cardiac parameters when performing an activity using a personalized cardiovascular hemodynamic model, in accordance with some embodiments of the present disclosure.

FIG.2 illustrates an exemplary block diagram of a cardiovascular hemodynamic model, in accordance with some embodiments of the present disclosure.

FIG.3A through FIG.3B illustrates an exemplary flow diagram of a computer implemented method for estimating cardiac parameters when performing an activity using a personalized cardiovascular hemodynamic model, in accordance with some embodiments of the present disclosure.

FIG.4 illustrates an exemplary block diagram of the autoregulation method for estimating unstressed blood volume, in accordance with some embodiments of the present disclosure.

FIG.5 illustrates heart rate estimation from a Photoplethysmogram (PPG) signal and an accelerometer signal, in accordance with some embodiments of the present disclosure.

FIG.6 illustrates an input-output relation of the personalized cardiovascular hemodynamic model, when an activity is performed by the subject, in accordance with some embodiments of the present disclosure.

FIG.7 illustrates estimation of Systemic Vascular Resistance (SVR) of the lower body of a subject, based on Metabolic EquivalenT (MET) levels, in accordance with some embodiments of the present disclosure.

FIG.8 illustrates estimated heart rate from a raw electrocardiogram (ECG) signal for a subject 11, in accordance with some embodiments of the present disclosure.

FIG.9 illustrates heart rate in the form of a box plot for the subject 11, in accordance with some embodiments of the present disclosure.

FIG.10 illustrates cardiac output in the form of a box plot for the subject 11, in accordance with some embodiments of the present disclosure.

FIG.11 illustrates ejection fraction of a box plot for the subject 11, in accordance with some embodiments of the present disclosure.

FIG.12 illustrates mean arterial pressure in the form of a box plot for the subject 11, in accordance with some embodiments of the present disclosure.

FIG.13 illustrates estimated MET using a vertical accelerometer signal for a subject 2, in accordance with some embodiment of the present disclosure.

FIG.14 illustrates estimated SVR using the estimated MET for the subject 2, in accordance with some embodiment of the present disclosure.

FIG.15 illustrates estimated heart rate from PPG signal for the subject 2, in accordance with some embodiment of the present disclosure.

FIG.16 illustrates estimated unstressed blood volume based on the autoregulation method for the subject 2, in accordance with some embodiment of the present disclosure.

FIG.17 illustrates estimated heart rate from PPG signal in the form of a box plot for the subject 2, in accordance with some embodiments of the present disclosure.

FIG.18 illustrates estimated cardiac output in the form of a box plot for the subject 2, in accordance with some embodiments of the present disclosure.

FIG.19 illustrates estimated ejection fraction in the form of a box plot for the subject 2, in accordance with some embodiments of the present disclosure.

FIG.20 illustrates estimated mean arterial pressure in the form of a box plot for the subject 2, in accordance with some embodiments of the present disclosure.

DETAILED DESCRIPTION OF EMBODIMENTS

Exemplary embodiments are described with reference to the accompanying drawings. In the figures, the left-most digit(s) of a reference number identifies the figure in which the reference number first appears. Wherever convenient, the same reference numbers are used throughout the drawings to refer to the same or like parts. While examples and features of disclosed principles are described herein, modifications, adaptations, and other implementations are possible without departing from the scope of the disclosed embodiments.

Considering the risks posed by Cardiovascular disease (CVD), it is very important to periodically monitor a patient's cardiac parameters to assess effects of prescribed exercises on the cardiac parameters of the patient. Unless the patient diligently visits a caregiver, say during a post-operative period, assessing cardiovascular conditions for recovery is challenging. A lapse on the part of the patient may be detrimental to the patient's health. The present disclosure enables personalized cardiac rehabilitation guidance and care continuum using a personalized cardiovascular hemodynamic model that effectively simulates cardiac parameters when the patient performs an activity using a wearable device like a digital watch that can help capture Electrocardiogram (ECG) signal, Photoplethysmogram (PPG) signal and accelerometer signal. In the context of the present disclosure, the expression 'patient' may interchangeably be referred as a 'subject'.

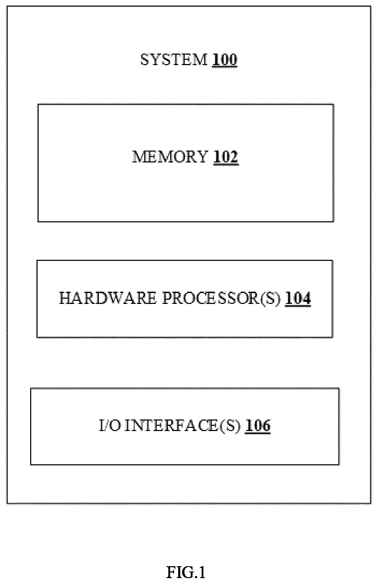

Referring now to the drawings, and more particularly to FIG. 1 through FIG.20, where similar reference characters denote corresponding features consistently throughout the figures, there are shown preferred embodiments and these embodiments are described in the context of the following exemplary system and/or method.

FIG.1 illustrates an exemplary block diagram of a system 100 for estimating cardiac parameters when performing an activity using a personalized cardiovascular hemodynamic model, in accordance with some embodiments of the present disclosure. In an embodiment, the system 100 includes one or more hardware processors 104, communication interface device(s) or input/output (I/O) interface(s) 106, and one or more data storage devices or memory 102 operatively coupled to the one or more hardware processors 104. The one or more hardware processors 104 can be implemented as one or more microprocessors, microcomputers, microcontrollers, digital signal processors, central processing units, state machines, graphics controllers, logic circuitries, and/or any devices that manipulate signals based on operational instructions. Among other capabilities, the processor(s) are configured to fetch and execute computer-readable instructions stored in the memory. In the context of the present disclosure, the expressions 'processors' and 'hardware processors' may be used interchangeably. In an embodiment, the system 100 can be implemented in a variety of computing systems, such as laptop computers, notebooks, hand-held devices, workstations, mainframe computers, servers, a network cloud and the like.

I/O interface(s) 106 can include a variety of software and hardware interfaces, for example, a web interface, a graphical user interface, and the like and can facilitate multiple communications within a wide variety of networks N/W and protocol types, including wired networks, for example, LAN, cable, etc., and wireless networks, such as WLAN, cellular, or satellite. In an embodiment, the I/O interface(s) can include one or more ports for connecting a number of devices to one another or to another server.

The memory 102 may include any computer-readable medium known in the art including, for example, volatile memory, such as static random access memory (SRAM) and dynamic random access memory (DRAM), and/or non-volatile memory, such as read only memory (ROM), erasable programmable ROM, flash memories, hard disks, optical disks, and magnetic tapes. In an embodiment, one or more modules (not shown) of the system 100 can be stored in the memory 102.

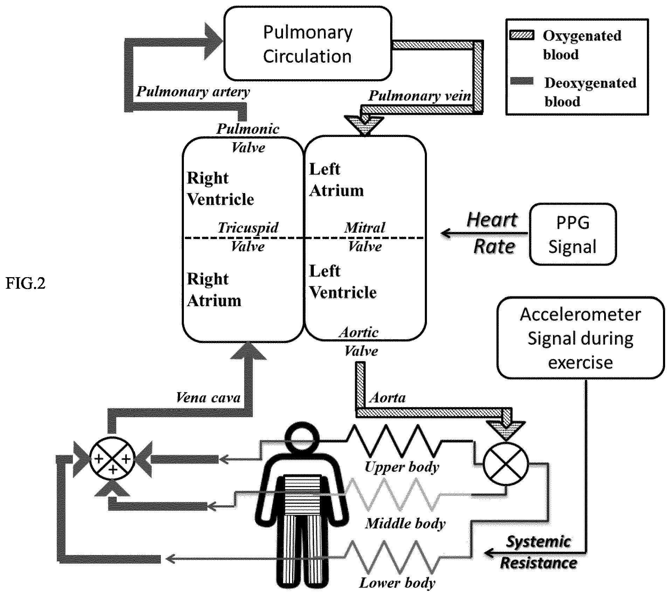

FIG.2 illustrates an exemplary block diagram of a cardiovascular hemodynamic model triggered via activity related data, in accordance with some embodiments of the present disclosure. Heart is a muscular organ in which each half is composed of a pair of atria and a pair of ventricles serving like a pulsatile pump. A left heart chamber, comprising a left ventricle (lv) and a left atrium (la), pumps oxygenated blood to all tissues of the body. This specific circulation is called systemic circulation. On the other hand, a right heart, comprising a right ventricle (rv) and a right atrium (ra), drives deoxygenated blood to the lungs forming a pulmonic circulation. In addition, there are four cardiac valves namely, mitral (mi) and aortic (ao) valves in the left heart and tricuspid (tr) and pulmonic (pu) valves in the right heart respectively. These valves synchronously open and close based on a pressure difference within the cardiac chambers and ensures rhythmic unidirectional flow through the heart.

In accordance with the present disclosure, following assumptions have been considered to describe the hemodynamics of the cardiac system.

Assumption 1: Each of the cardiac chambers is triggered by an autonomous compliance function (C(t)), due to the elasticity of the cardiac walls. So, the volume (V (t)), at time t, across any cardiac chamber is defined as V (t) = C(t) × P (t) + Vs; where P (t) is the pressure at the t$^{th}$ time and Vs is the unstressed volume of that particular chamber. Assumption 2: The cardiac chambers are considered compliance vessels. Hence, the rate of change of volume across a cardiac chamber at time t, is defined as a difference between an inflow Q1(t) and an outflow Q2(t), so, dV/dt (t) = Q1(t) - Q2(t). Assumption 3: Each vessel is considered a resistive vessel, as the blood flow is impeded due to frictional forces, depending on viscosity of blood, diameter of the blood vessels, etc. Thus, a flow across a resistive vessel is Q(t) = ΔP(t)/R; where ΔP(t) is a pressure difference in successive compartments of the vessel, at time t and R is the vascular resistance of that vessel.

Based on the three assumptions provided herein above, pressure dynamics of a cardiovascular hemodynamic model is analytically defined as:

$$\dot{P}_{la} = \frac{1}{C_{la}(t)}\left[\frac{P_{la} - P_{pa}}{R_p} - U_{mi} \times \frac{P_{la} - P_{lv}}{R_{mi}} - \dot{C}_{la}(t)P_{la}\right]$$

$$\dot{P}_{lv} = \frac{1}{C_{lv}(t)}\left[U_{mi} \times \frac{P_{la} - P_{lv}}{R_{mi}} - U_{ao} \times \frac{P_{lv} - P_{sa}}{R_{ao}}\dot{C}_{lv}(t)P_{lv}\right]$$

$$\dot{P}_{sa} = \frac{1}{C_{sa}}\left[U_{ao} \times \frac{P_{lv} - P_{sa}}{R_{ao}} - \frac{P_{sa} - P_{ra}}{R_s}\right]$$

$$\dot{P}_{ra} = \frac{1}{C_{ra}(t)}\left[\frac{P_{sa} - P_{ra}}{R_s} - U_{tr} \times \frac{P_{ra} - P_{rv}}{R_{tr}} - \dot{C}_{ra}(t)P_{ra}\right]$$

$$\dot{P}_{rv} = \frac{1}{C_{rv}(t)}\left[U_{tr} \times \frac{P_{ra} - P_{rv}}{R_{tr}} - U_{pu} \times \frac{P_{rv} - P_{pa}}{R_{pu}}\dot{C}_{rv}(t)P_{rv}\right]$$

$$\dot{P}_{pa} = \frac{1}{C_{pa}}\left[U_{pu} \times \frac{P_{rv} - P_{pa}}{R_{pu}} - \frac{P_{la} - P_{pa}}{R_p}\right]$$

➔ (1)

where $\dot{P}_{la}$, $\dot{P}_{lv}$, $\dot{P}_{sa}$, $\dot{P}_{ra}$, $\dot{P}_{rv}$ and $\dot{P}_{pa}$ are pressure variables in the *la, lv*, systemic arteries *sa, ra, rv* and pulmonary arteries pa respectively, having initial conditions of $p_{la}^0$, $p_{lv}^0$, $p_{sa}^0$, $p_{ra}^0$, $p_{rv}^0$ and $p_{pa}^0$. The valvular resistance across the mitral, aortic, tricuspid and pulmonic valves are $R_{mi}$, $R_{ao}$, $R_{tr}$ and $R_{pu}$ respectively. The vascular resistance and compliance pair, across the pulmonic and systemic vessels are $R_p$, $C_{pa}$ and $R_s$, $C_{sa}$ respectively.

$U_i; \forall\{mi,ao,tr,pu\}$ are inputs for opening and closing of the heart valves as mentioned in equation (2) below.

$$U_{mi} = \begin{cases} 1, & if, P_{la} > P_{lv}, \\ \delta_{mi}, & otherwise \end{cases};$$

$$U_{ao} = \begin{cases} 1, & if, P_{lv} > P_{sa} \\ \delta_{ao}, & otherwise \end{cases};$$

$$U_{tr} = \begin{cases} 1, & if, P_{ra} > P_{rv}, \\ \delta_{tr}, & otherwise \end{cases};$$

$$U_{pu} = \begin{cases} 1, & if, P_{rv} > P_{pa} \\ \delta_{pu}, & otherwise \end{cases},$$

where $\delta_{mi}$, $\delta_{ao}$, $\delta_{tr}$, and $\delta_{pu}$ define regurgitation effect of the cardiovascular system.

➜ (2)

As per the assumption 1, the cardiac chambers are activated sequentially, in a synchronized manner, by time-varying compliance functions. Typically, this activation starts from a sinoatrial node, which is located inside *ra*, then, it traverses to the *la* with a time delay of $d_{la}$ , causing them to contract for pumping the blood into the ventricles. After that, the activation traverses from the atrium to the ventricles via an atrioventricular node with a time delay of *d*, allowing the ventricles to fill with blood. In accordance with the present disclosure, the compliance functions $C_{ra}(t)$, $C_{la}(t)$, $C_{lv}(t)$ and $C_{rv}(t)$ to sequentially actuate *ra, la, rv* and *lv* are defined as given in equation (3), equation (4) and equation (5) respectively.

$$C_{ra}(t) = C_{min,ra} + 0.5 \times (C_{max,ra} - C_{min,ra})u(t),$$

$$\text{wherein } u(t) = \begin{cases} 0, & 0 \le t < T_a \\ 1 - cos\left(2\pi\frac{t-T_a}{T-T_a}\right), & T_a \le t < T \end{cases}$$

➜ (3)

where $C_{min,ra}$ and $C_{max,ra}$ are the minimum and maximum values of the *ra* compliance and $u(t)$ is the activation function. The time *t* is considered over a complete cardiac cycle. $T_a$ is the start of the activation of *ra* and *T* is the end of the cardiac cycle.

Similarly, in accordance with the present disclosure, the compliance function to actuate *la* is modeled using equation (3), where $C_{min,la}$ and $C_{max,la}$ are the minimum and maximum values of the *la* compliance, $u(t)$ is the activation function of *la* and $d_{la}$ represent a delay in activation of *la* with respect to *ra*.

$$C_{la}(t) = C_{min,la} + 0.5 \times (C_{max,la} - C_{min,la})u(t - d_{la})$$

➜ (4)

Likewise, in accordance with the present disclosure, the compliance functions to actuate *rv* and *lv* are represented by equation (5) below, where $C_i$; *i* E {*lv,rv*} is the systolic compliance across *lv* or *rv* and is estimated as a ratio of R-peak and T-peak of the ECG signal, $u_v(t)$ is the activation function, and *d* represents the delay in activation of *lv* or *rv* from *ra*. $T_1$ and $T_2$ are the systolic and diastolic activation time instances of the cardiac cycle respectively.

$$C_i(t) = C_i \times u_v(t - d), i \in \{lv, rv\},$$

$$\text{wherein } u_v(t) = \begin{cases} 0.5 - 0.5cos\left(\pi\frac{1}{T_1}\right), & 0 \le t < T_1 \\ 0.5 + 0.5cos\left(\pi\frac{t-T_1}{T_2-T_1}\right), & T_1 \le t < T_2 \\ 0, & T_2 \le t < T \end{cases}$$

➜ (5)

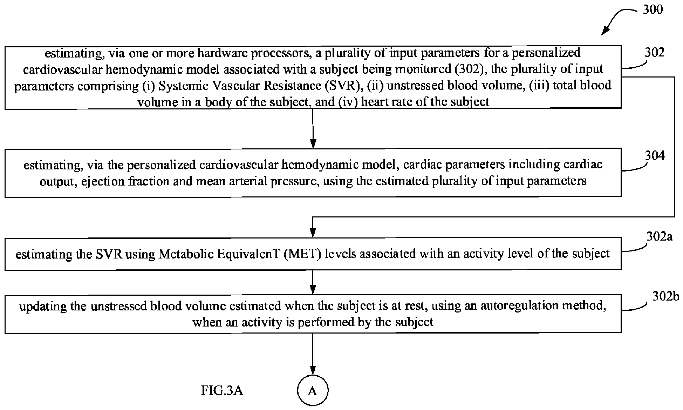

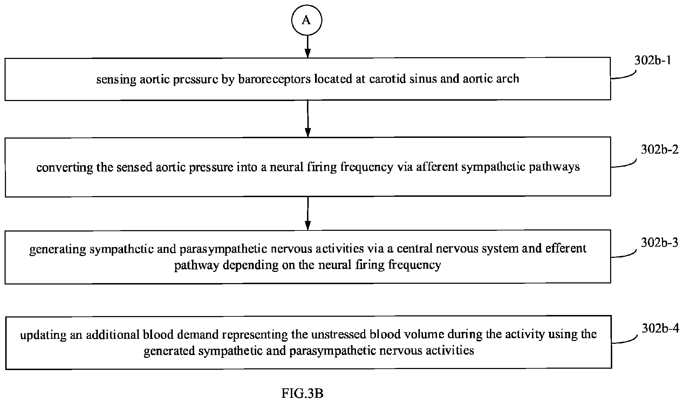

In accordance with the present disclosure, depending on pressure variations in the cardiac chambers, blood circulates as shown in FIG.3. Based on the circulation, the volume across the cardiac chambers also get developed as per assumption 2 mentioned above. The rate of change of volume across the cardiac chambers is measured as given in equation (6) below.

$$\dot{V}_i = C_i \dot{P}_i + \dot{C}_i P_i; \forall i \in \{la, lv, ra, rv\}$$

➔ (6)

Similarly, the rate of change of volume across the systemic and pulmonary arteries is evaluated using equation (7) below.

$$\dot{V}_j = C_j \dot{P}_j; \forall j \in \{sa, pa\}$$

➔ (7)

The overall blood volume using equations (6) and (7) above is represented by equation (8) below.

$$V_{total} = \sum_i V_i + \sum_j V_j$$

➔ (8)

FIG.3A through FIG.3B illustrate an exemplary flow diagram of a computer implemented method 300 for estimating cardiac parameters when performing an activity using a personalized cardiovascular hemodynamic model, in accordance with some embodiments of the present disclosure. In an embodiment, the system 100 includes one or more data storage devices or memory 102 operatively coupled to the one or more hardware processors 104 and is configured to store instructions configured for execution of steps of the method 200 by the one or more hardware processors 104. The steps of the method 300 will now be explained in detail with reference to the components of the system 100 of FIG.1. Although process steps, method steps, techniques or the like may be described in a sequential order, such processes, methods and techniques may be configured to work in alternate orders. In other words, any sequence or order of steps that may be described does not necessarily indicate a requirement that the steps be performed in that order. The steps of processes described herein may be performed in any order practical. Further, some steps may be performed simultaneously.

Accordingly, in an embodiment of the present disclosure, the one or more hardware processors 104, are configured to estimate, at step 302, a plurality of input parameters for a personalized cardiovascular hemodynamic model associated with a subject being monitored. In an embodiment, the plurality of input parameters comprises (i) Systemic Vascular Resistance (SVR), (ii) unstressed blood volume, (iii) total blood volume in a body of the subject, and (iv) heart rate of the subject.

When the subject performs any activity like say physical exercise, the amount of blood flowing through skeletal muscles is significantly increased and it is solely dependent on the intensity of the activity. In accordance with the present disclosure, systemic vessels are considered as resistive vessels. Therefore, to accommodate increasing blood flow across the systemic vessels, the vessel resistivity requires to be reduced that significantly reduces the resistance across the systemic vessels. In accordance with the present disclosure, to estimate how much the vessel resistivity needs to be changed and how it is correlated with the level of exercise, the metabolic rate of the subject (which defines the rate of change energy expended per unit time) is considered as it is tightly coupled with vessel conductance.

For a specific activity, the metabolic rate is measured by a metabolic equivalent of task or Metabolic EquivalenT (MET) levels. The MET is defined as the ratio of an exercise metabolic rate to a resting metabolic rate. Thus, for a given activity, the MET value has been considered a constant. For example, in light intensive activities, such as walking slowly, the MET level is consider to be less than 3, in moderate intensive activities (walking at say 4.5 km/hr), the MET level is considered between 3 and 6 and for intensive exercises, such as jogging at say 11 km/hr, the MET level is greater than 6.

Thus, in accordance with the present disclosure, the one or more hardware processors 104, are configured to estimate, at step 302a, the SVR using the MET levels associated with an activity level of the subject. In an embodiment, the SVR is represented as given in equation (9) below.

$$R_s(t) = \begin{cases} R_s(0) & \text{if } MET(t) < 2.5 \\ \dfrac{R_s(0)}{MET(t)} * e^{-\frac{t*MET(t)}{\tau}} & \text{otherwise} \end{cases}$$ , wherein $R_s(0)$ represents the SVR at rest, $MET(t)$ represents a metabolic equivalent of the activity performed at the $t^{th}$ time, and $\tau$ is a time constant.

➜ (9)

In order to simulate the effect of the activity with the variation of systemic resistance, the systemic circulation part in a body is subdivided into three generic sections such as an upper body, a middle body and a lower body as shown in FIG.2. Depending on the activity performed by the subject, the SVR across the section is modulated based on the MET level as described above, and the SVR in the other sections are assumed to be constant or modulated by an autoregulation method described hereinafter.

Unstressed blood volume (or dead volume) is defined as the maximum volume of blood that can be placed inside a capacitive vessel without raising its effective pressure above 0 mmHg. As per literature, in normal cardiac condition (resting state), the unstressed blood volume is approximated as shown in equation (10) below.

$$V_s = 0.6 * V_{total}$$

➜ (10)

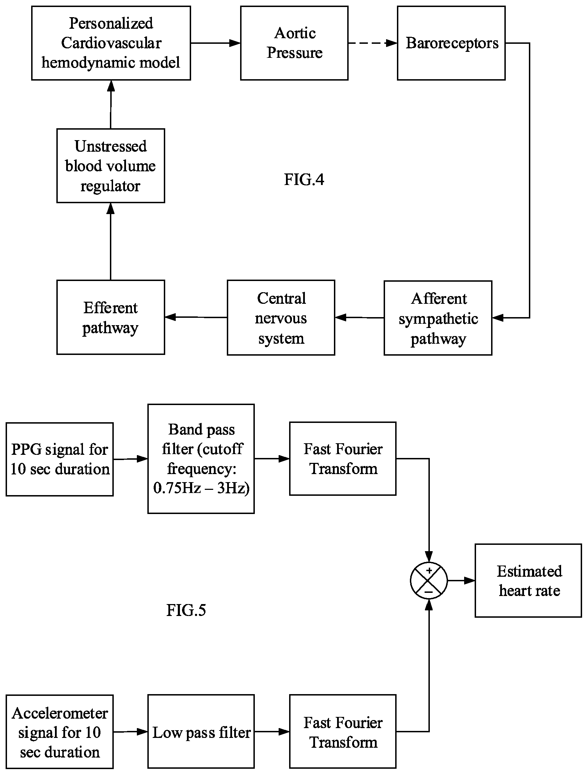

Therefore, in a resting state around 40% of the total blood volume ($V_{total}$) is stressed volume (effectively participate in hemodynamics) and the rest is unstressed volume. When the subject performs an activity, or during an exercise condition, because of the increasing demand of blood, a portion of the unstressed blood is added into the stressed volume. Baroreflex regulates the effect of unstressed blood volume while enhancing the venous return of blood during a ventricular systolic phase. FIG.4 illustrates an exemplary block diagram of the autoregulation method for estimating unstressed blood volume, in accordance with some embodiments of the present disclosure. In an embodiment of the present disclosure, the one or more hardware processors 104, are configured to update, at step 302b, the unstressed blood volume estimated at rest (from the total blood volume - refer equation 10) using the autoregulation method, when an activity is performed by the subject. As seen in FIG.4, four subsystems, viz., an afferent sympathetic pathway, central nervous system, efferent pathway and unstressed blood volume regulator cooperate to enable autoregulation.

The effect of baroreflex starts from sensing of aortic pressure by baroreceptors located at carotid sinus and aortic arch. Accordingly, in an embodiment of the present disclosure, the autoregulation method comprises sensing aortic pressure by baroreceptors located at carotid sinus and aortic arch, at step 302b-1. The sensed aortic pressure is converted into a neural firing frequency $f_{aff}(P)$ via afferent sympathetic pathways, at step 302b-2. Analytically, this functionality is defined as shown in equation (11) below.

$$f_{aff}(P) = \frac{f_{min} + f_{max} * \exp\left(\frac{P}{K_a}\right)}{1 + \exp\left(\frac{P}{K_a}\right)}$$ , where $P$ defines the sensed aortic pressure, $f_{min}$ and

$f_{max}$ are the upper and lower saturation frequencies, $K_a$ is a constant parameter having the same unit as pressure.

➜ (11)

Sympathetic and parasympathetic nervous activities are then generated via the central nervous system and efferent pathway, depending on the neural firing frequency, at step 302b-3. The sympathetic activity $f_{sa}$ is defined as shown in equation (12) below.

$$f_{sa} = f_{sa\infty} + (f_{sa0} - f_{sa\infty}) \exp\left(-K_{sa} * f_{aff}\right), \text{ where } K_{sa}, f_{sa\infty} \text{ and } f_{sa0}$$
➜ (12)

are constants. The Parasympathetic activity $f_{psa}$ is defined as shown in equation (13) below.

$$f_{psa} = \frac{f_{psa0} + f_{psa\infty} * \exp\left(\frac{f_{aff}}{K_{psa}}\right)}{1 + \exp\left(\frac{f_{aff}}{K_{psa}}\right)}, \text{ where } K_{psa}, f_{psa\infty} \text{ and } f_{psa0} \text{ are constants.}$$

$$\rightarrow (13)$$

Based on the generated sympathetic and parasympathetic nervous activities, the additional blood volume requirement during an exercise needs to be regulated. Autoregulation of the unstressed blood volume via the unstressed blood volume regulator is analytically represented as shown in equation (14) below.

$$V_s(t) = V_s(0) + \Delta V_s^{sa}(t) + \Delta V_s^{psa}(t), \text{ where } V_s(0) \text{ defines the unstressed blood}$$

volume at rest, $\Delta V_s^{sa}(t)$ and $\Delta V_s^{psa}(t)$ are the change in unstressed blood volume due

to sympathetic and parasympathetic activities.

$$\rightarrow (14)$$

The governing equations of $\Delta V_s^{sa}(t)$ and $\Delta V_s^{psa}(t)$ are defined as given in equations (15) and (16) below.

$$\frac{\delta \Delta V_s^{sa}(t)}{\delta t} = -\frac{\Delta V_s^{sa}(t)}{\tau^{sa}} + K * \ln(1 + f_{sa} - f_{sa0}),$$

$$\rightarrow (15)$$

$$\frac{\delta \Delta V_s^{psa}(t)}{\delta t} = -\frac{\Delta V_s^{psa}(t)}{\tau^{psa}} + K * f_{psa},$$

$$\rightarrow (16)$$

where $K$ is a constant, and $\tau^{sa}$, $\tau^{psa}$ are the time constant of the sympathetic and parasympathetic activities respectively.
An additional blood demand representing the unstressed blood volume during the activity, is then updated using the generated sympathetic and parasympathetic nervous activities, at step 302b-4.
In accordance with the present disclosure, the total blood volume which is part of the plurality of input parameters is estimated using height and weight of the subject. As per medical literature, the total blood volume across a healthy human body (who does not have any kind diseases) is closely related with Body-Surface-Area (BSA), and the BSA correlates with the height h and the weight w of the subject. Accordingly, the total blood volume is defined as shown in equation (17) below.

$$V_{total} = 3.29 \times BSA - 1.229, \text{ where } BSA = \sqrt{\frac{h \times w}{3600}}$$

$$\rightarrow (17)$$

The heart rate is an important parameter that dynamically changes during exercise. Hence, creating a model of heart rate based on a specific physical activity is very difficult to achieve. In accordance with the present disclosure, the personalized cardiovascular hemodynamic model is executed considering the heart rate is continuously monitored when the subject performs an activity. In accordance with the present disclosure, the heart rate of the subject, which is part of the plurality of input parameters, is estimated using (i) a Photoplethysmogram (PPG) signal and an accelerometer signal or (ii) an ECG signal, from a wearable device, such as a digital watch, worn by the subject when performing the activity. As per literature, noise effect of an ECG signal during activities is less than the PPG signal. Hence, if the ECG signal is available, the heart rate is easily measured continuously using detected peaks. If only the PPG signal is available, then the heart rate for each cycle may not be obtained. Hence, the heart rate for a specific time-window is estimated by employing noise cancellation technique using the accelerometer signal. In an embodiment, the accelerometer signal is a 3-axis accelerometer signal, also captured continuously via the wearable device. FIG.5 illustrates heart rate estimation from a PPG signal and an accelerometer signal, in accordance with some embodiments of the present disclosure. Fast Fourier transform is performed on the PPG signal and the accelerometer signal in parallel for the same duration. Heart rate is estimated based on peaks available in the PPG signal after discarding the peaks (representing noise) obtained from the accelerometer signal.

In an embodiment, the step of estimating a plurality of input parameters is preceded by personalizing a cardiovascular hemodynamic model to obtain the personalized cardiovascular hemodynamic model. In an embodiment, the personalizing is based on one or more of (i) cardiac parameters obtained from an echocardiogram such as valvular area, (ii) ECG signal obtained from a wearable device worn by the subject when performing the activity, and (iii) metadata of the subject including height and weight associated thereof. In accordance with the present disclosure, the cardiovascular hemodynamic model is configured with the compliance functions $C_{ra}(t)$, $C_{la}(t)$, $C_{lv}(t)$ and $C_{rv}(t)$ to sequentially actuate *ra, la, rv* and *lv* as explained above. Echocardiography information of the subject helps to derive constant parameters in equation (1) above. For instance, the mitral valve resistance $R_{mi}$, the aortic valve resistance $R_{ao}$, the tricuspid valve resistance $R_{tr}$ and the pulmonic valve resistance $R_{pu}$ may be accurately calculated from the echocardiography information, more specifically from information pertaining to valvular diameter of the subject, thereby personalizing the cardiovascular hemodynamic model. Again, the parameters $\delta_{mi}$, $\delta_{ao}$, $\delta_{tr}$ and $\delta_{pu}$ in equation (2) above, that define the regurgitation effect may be estimated from the echocardiography information. Furthermore, the parameters $T$ and $T_a$ in equation (3) above, and $T_1$, $T_2$ and $d$ in equation (5) above may be estimated based on an ECG signal from the subject obtained using a wearable device. Metadata pertaining to the subject such as the height and the weight of the subject may be used to compute the total blood volume using equation (17) above.

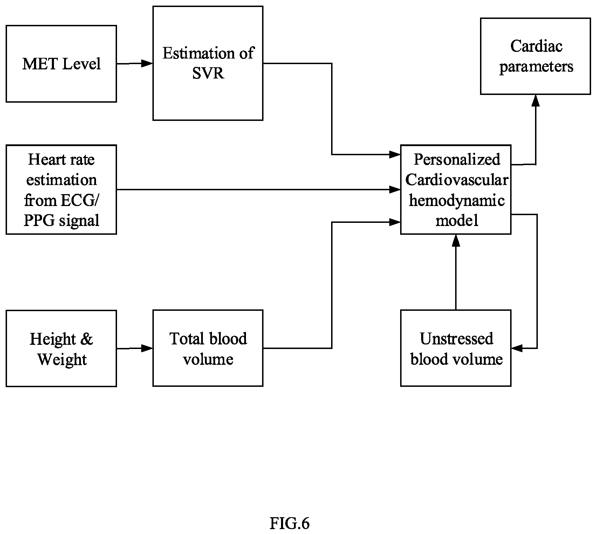

In an embodiment of the present disclosure, at step 304, cardiac parameters are estimated via the personalized cardiovascular hemodynamic model, using the estimated plurality of input parameters estimated at step 302. In an embodiment, the cardiac parameters include cardiac output, ejection fraction and mean arterial pressure. FIG.6 illustrates an input-output relation of the personalized cardiovascular hemodynamic model, when an activity is performed by the subject, in accordance with some embodiments of the present disclosure.

SIMULATION RESULTS AND DISCUSSION

Experiment 1: Publicly available Troika dataset was used. In these experiments, 12 subjects in the age group 18years to 35years were asked to run on a treadmill with changing speeds. The speed-variations are defined as follows. Rest for 30sec → 6-8km/hr for 1 min → 12-15km/hr for 1min → 6-8km/hr for 1 min → 12-15km/hr for 1 min → Rest for 30sec. While performing exercises on the treadmill, two-channel PPG signals, three-axis acceleration signals, and one-channel ECG signals were simultaneously recorded from each subject. For each subject, the PPG signals were recorded from the wrist by two pulse oximeters with green LEDs (wavelength: 515nm). Their distance (from center to center) was 2 cm. The acceleration signal was also recorded from the wrist by a three-axis accelerometer. Both the pulse oximeter and the accelerometer were embedded in a wristband, worn by the subjects. The ECG signal was recorded simultaneously from the chest using wet ECG sensors. All signals were sampled at 125 Hz and sent to a computer via Bluetooth.

The cardiovascular hemodynamic model of the present disclosure was provided with the plurality of input parameters as described hereinafter. Total blood volume: Subject specific metadata in the form of height and weight is not available in the Troika dataset. Hence, it was assumed that all the subjects are healthy, and the total blood volume was assumed to be 5 liters for each subject.

SVR during exercise: In the Troika dataset, the level of exercise has been clearly mentioned; hence, the respective MET levels (value) were predicted accurately from the MET table. The MET values for the respective activity duration are tabulated below in Table 1.

Table 1: MET table with respect to speed variation

| Activity duration (sec) | Running Speed (km/hr) | MET Value |
| --- | --- | --- |
| 0-30 | Rest | 2 |
| 31-90 | 6-8 | 8 |
| 91-150 | 12-15 | 15 |
| 151-210 | 6-8 | 8 |
| 211-270 | 12-15 | 15 |
| 271-300 | Rest | 2 |

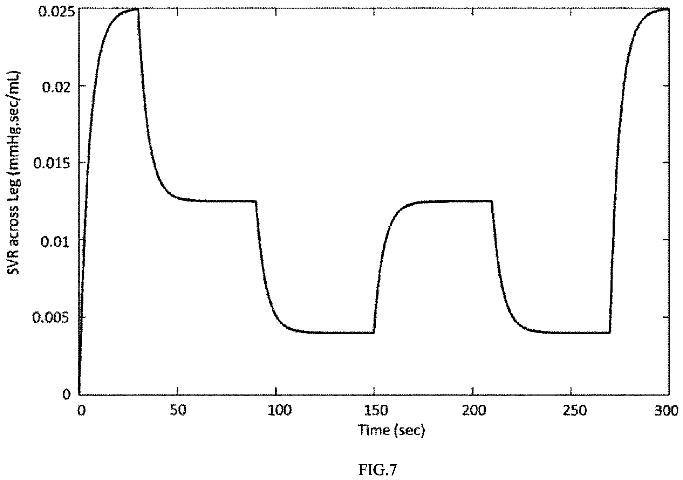

Depending on the MET values, the estimated SVR across the lower body was estimated using equation (9) above. FIG.7 illustrates estimation of the SVR of the lower body of a subject, based on MET levels, in accordance with some embodiments of the present disclosure. In this simulation, the value of $R_s(0)$ which defines the SVR at the resting state, was chosen as 0.05 mmHgsec/mL and the time constant $\tau$ was 0.5 sec.

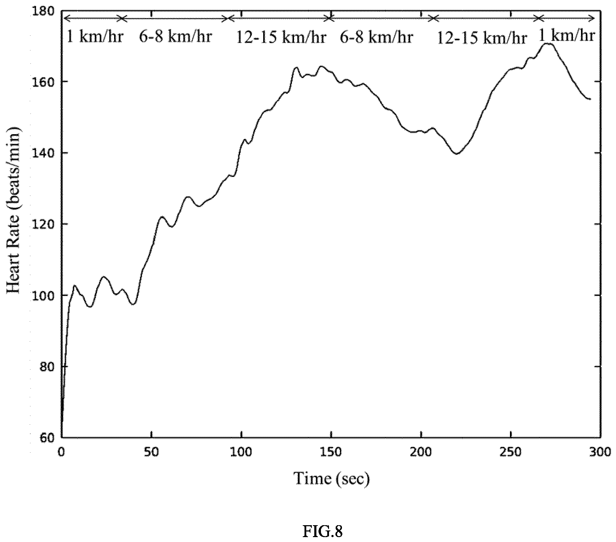

Heart rate estimation from ECG signal: In the Troika dataset, as the ECG signal has been captured during the physical activities, by filtering and detecting the ECG peaks, the continuous heart rate was estimated. FIG.8 illustrates estimated heart rate from a raw ECG signal for a subject 11 (of the 11 subjects considered, the ECG signal from the 12th subject being erroneous), in accordance with some embodiments of the present disclosure. Unstressed blood volume is approximated using the autoregulation method.

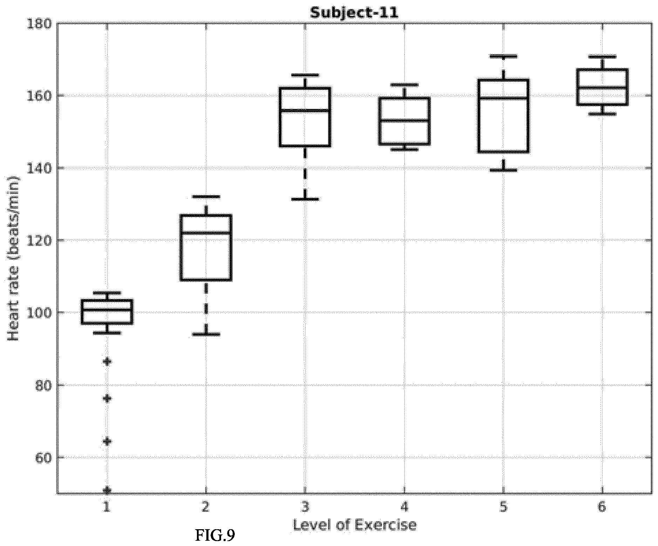

By inputting the plurality of input parameters mentioned above into the cardiovascular hemodynamic model of the present disclosure, the cardiac parameters such as the cardiac output, the ejection fraction and the mean arterial pressure were estimated. FIG.9 illustrates heart rate in the form of a box plot for the subject 11, in accordance with some embodiments of the present disclosure, wherein the heart rate was estimated using the ECG signal. Similar box plots were derived for all the 11 subjects considered. Table 2 below provides a comparison of the estimated heart rate at different levels of physical activities for the 11 subjects.

Table 2: Heart rate comparison

| Subject Number | Levels of physical activities of Troika Dataset | | | | | |
|---|---|---|---|---|---|---|
| | Level 1 (Rest for 30 sec.) | Level 2 (Running @6-8 km/hr for 60 sec.) | Level 3 (Running @12-15 km/hr for 60 sec.) | Level 4 (Running @6-8 km/hr for 60 sec.) | Level 5 (Running @12-15 km/hr for 60 sec.) | Level 6 (Rest for 30 sec.) |
| 1 | 72.84 + 10.14 | 100.16 + 12.71 | 139.95 + 11.21 | 153.72 + 1.86 | 157.68 + 5.75 | 160.98 ± 4.27 |
| 2 | 78.64 + 13.7 | 99.62 + 11.1 | 128.65 ± 9.26 | 133.26 ± 8.26 | 134.63 ± 8.82 | 143.84 ± 4.68 |
| 3 | 94.82 + 12.04 | 108.22 + 10.39 | 143.27 ± 9.35 | 142.98 + 8.12 | 145.4 + 10.31 | 158.60 ± 1.8 |
| 4 | 82.46 + 10.52 | 112.25 + 10.45 | 142.16 + 8.46 | 142.52 + 5.58 | 154 + 10.41 | 150.38 + 5.55 |
| 5 | 106.11 + 10.36 | 120.48 + 9.05 | 149.60 + 9.27 | 153.29 ± 8 | 154.86 + 8.25 | 161.52 ± 3.32 |
| 6 | 70.49 + 10.62 | 113.99 + 13.93 | 143.64 + 7.55 | 143.69 ± 6.97 | 144.61 + 6.6 | 150.51 ± 3.05 |
| 7 | 93.02 + 9.98 | 113.34 + 7.52 | 142.4 ± 9.15 | 146.17 + 3.23 | 151.4 + 5.98 | 154.75 + 2.34 |
| 8 | 77.59 + 11.55 | 113.83 + 10.59 | 139.21 ± 11.34 | 140.83 + 7.42 | 135.54 + 7.34 | 124.62 + 1.87 |
| 9 | 78.60 + 9.91 | 103.14 ± 9.54 | 134.9 + 12.22 | 140.27 ± 6.65 | 140.01 ± 7.66 | 146.91 + 4.77 |
| 10 | 124.62 + 13.42 | 150.00 ± 9.99 | 166.47 ± 4.03 | 162.98 + 4.02 | 172.93 ± 4.29 | 173.40 + 2.79 |
| 11 | 97.94 + 10.45 | 118.26 + 10.74 | 153.4 + 10.03 | 153.26 + 5.98 | 155.89 + 10.48 | 162.61 + 5.54 |

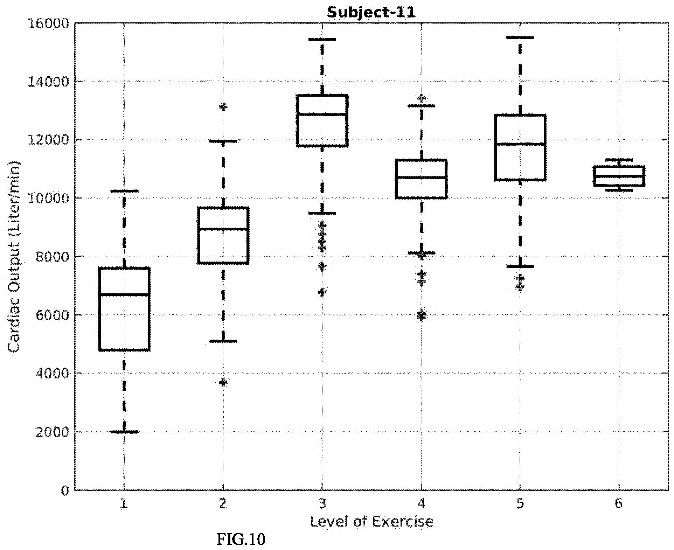

FIG.10 illustrates cardiac output in the form of a box plots for the subject 11, in accordance with some embodiments of the present disclosure. Similar box plots were derived for all the 11 subjects considered. Table 3 below provides a comparison of the estimated cardiac output at different levels of physical activities for the 11 subjects.

Table 3: Cardiac output comparison

| Subject Number | Levels of physical activities of Troika Dataset | | | | | |
|---|---|---|---|---|---|---|
| | Level 1 (Rest for 30 sec.) | Level 2 (Running @6-8 km/hr for 60 sec.) | Level 3 (Running @12-15 km/hr for 60 sec.) | Level 4 (Running @6-8 km/hr for 60 sec.) | Level 5 (Running @12-15 km/hr for 60 sec.) | Level 6 (Rest for 30 sec.) |
| 1 | 4.04 +2.08 | 7.23±1.9 | 14.4±2.13 | 11.5±2.13 | 13.4±2 | 10.8±2.25 |
| 2 | 4.7±2.17 | 7.38±1.92 | 13.36±1.6 | 9.4±1.6 | 11.2±1.6 | 12.07+0.4 |
| 3 | 4.6±2.6 | 6.64±1.86 | 9.51±1.85 | 8.8+1.85 | 8.9±2.6 | 7.8±0.09 |
| 4 | 4.5±2.5 | 7.8±1.6 | 10.36±1.45 | 9.91+1.45 | 10.2±1.81 | 11.35±0.42 |
| 5 | 5.42±2.7 | 7.66±1.7 | 10.1+1.33 | 8.09±1.33 | 8.7±1.6 | 8.4±0.17 |
| 6 | 4.53±1.7 | 7.52±1.6 | 11.6±1.19 | 10.13±1.18 | 10.5+1.36 | 10.1±0.23 |
| 7 | 6.35±2.7 | 9.5±1.94 | 12.9+1.54 | 11.77±1.54 | 12.7+1.74 | 12.7±0.19 |
| 8 | 4.6±2.01 | 7.9±2.04 | 10.3±1.18 | 8.69±1.2 | 8.8±1.13 | 8±0.5 |
| 9 | 6.9±2.8 | 9.5±1.95 | 13.87±1.84 | 12.43±1.8 | 13.2±1.92 | 13.8±0.45 |
| 10 | 6.5±2.2 | 10±1.55 | 14:t1.19 | 11.68±1.19 | 12.9±1.6 | 12.9±0.2 |
| 11 | 6.2±2.2 | 8.75±1.6 | 12.53±1.38 | 10.6±1.38 | 11.75±1.8 | 10.8±0.37 |

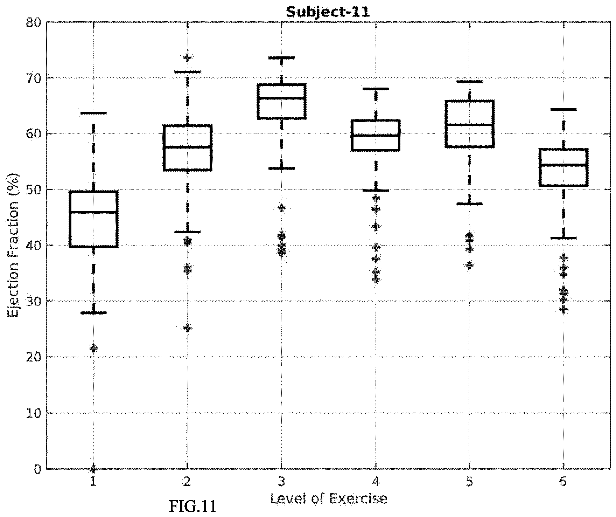

FIG.11 illustrates ejection fraction in the form of a box plot for the subject 11, in accordance with some embodiments of the present disclosure. Similar box plots were derived for all the 11 subjects considered. Table 4 below provides a comparison of the estimated ejection fraction at different levels of physical activities for the 11 subjects.

Table 4: Ejection fraction comparison

| Subject Number | Levels of ihvsical activities of Troika Dataset | | | | | |
|---|---|---|---|---|---|---|
| | Level 1 (Rest for 30 sec.) | Level 2 (Running @6-8 km/hr for 60 sec.) | Level 3 (Running @12-15 km/hr for 60 sec.) | Level 4 (Running @6-8 km/hr for 60 sec.) | Level 5 (Running @12-15 km/hr for 60 sec.) | Level 6 (Rest for 30 sec.) |
| 1 | 48.75±14.5 | 61.4±9.03 | 67.56±5.7 | 66.37±6.4 | 61.68±6.65 | 55.02±6.57 |
| 2 | 46.15±11.8 | 61.22±9.6 | 70±6.9 | 66.08±7.3 | 64.5±6.36 | 56.61±6.07 |
| 3 | 45.5±11.4 | 56.25±7.9 | 63.7±7.5 | 58.63±7.7 | 53.7±8.9 | 53.9±6.06 |
| 4 | 46.03±14.4 | 55.2±7.38 | 60.9±6.75 | 59.33±6.6 | 61.2±6.9 | 51.34±6.4 |
| 5 | 47.5±11.6 | 52.06±7.7 | 59.8±5.7 | 54.6±5.8 | 55.2±6 | 54.55±6.05 |
| 6 | 48.5±12.07 | 54.9±8.6 | 65.05±5.98 | 65.2±5.06 | 60.1±6.46 | 52.9±6.03 |
| 7 | 46.1±13.16 | 58.75±7.9 | 67.9±7.3 | 66.5±6.5 | 62.7±6.24 | 57.25±5.9 |
| 8 | 55.15±13.5 | 56.3±9.35 | 62.9±6.5 | 62.23±5.7 | 57.2±6.06 | 53.9±6.2 |
| 9 | 48.8±15.2 | 58.9±8.7 | 68.74±6.4 | 63.01±6.6 | 64.8±7.15 | 57.5±7.01 |
| 10 | 41.45±9.23 | 53.65±5.9 | 65.04±5.7 | 59.15±4.5 | 59.2±6.1 | 53.5±5.93 |
| 11 | 43.26±10.4 | 56.75±7.6 | 64.9±6.7 | 58.9±5.97 | 60.7±6.25 | 53.34±6.57 |

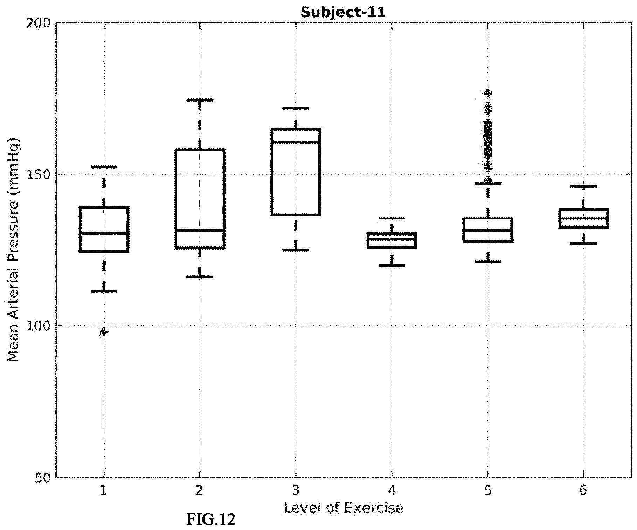

FIG.12 illustrates mean arterial pressure in the form of a box plot for the subject 11, in accordance with some embodiments of the present disclosure. Similar box plots were derived for all the 11 subjects considered. Table 5 below provides a comparison of the estimated mean arterial pressure at different levels of physical activities for the 11 subjects.

Table 5: Mean arterial pressure comparison.

| Subject Number | Levels of physical activities of Troika Dataset | | | | | |
|---|---|---|---|---|---|---|
| | Level 1 (Rest for 30 sec.) | Level 2 (Running @6-8 km/hr for 60 sec.) | Level 3 (Running @12-15 km/hr for 60 sec.) | Level 4 (Running @6-8 km/hr for 60 sec.) | Level 5 (Running @12-15km/hr for 60 sec.) | Level 6 (Rest for 30 sec.) |
| 1 | 99.85+27.2 | 129.54±14.3 | 144.38+18.5 | 126.7±12.9 9 | 138.57±13. 7 | 131.95±19.9 7 |
| 2 | 98.98±29.17 | 135.9±13.04 | 143.3±13.07 | 128.9±4.8 | 130.16±5.2 | 137.28±19.6 |
| 3 | 120.6+29.9 | 161.26±16.6 | 161.26+18.6 | 151.71±6.6 2 | 156.12+10. 5 | 150.47±21.1 2 |
| 4 | 92.5±27.8 | 135.75+12.5 | 136.13+16.0 3 | 139.42+3.6 | 127.08+3.7 | 136.71+11.9 |
| 5 | 123.5±26.44 | 163.14±14.6 | 152.6±15.6 | 140.9±2.94 | 143.01 +4.1 | 158.97±8.7 |
| 6 | 101.33+23.9 3 | 125.6+8.76 | 131.6±13.7 | 125.98±3.6 8 | 127.98±7.6 | 121.7±3.8 |
| 7 | 130.9+30.3 | 154.9±23.34 | 145.44+14.9 5 | 142.78±6.8 5 | 136.5±6.5 | 130.5+17.07 |
| 8 | 104.18+26.9 | 122.01±10.9 3 | 120.85±11.9 4 | 118.17±2.5 8 ± | 131.7+11.9 | 122.4+3.9 |
| 9 | 107.3±32.35 | 134.8± 15.45 | 149.04±15.5 | 137.14+6.5 4 | 139.25±7.7 3 | 149.7±19.8 |
| 10 | 133.4±25.7 | 141.1±8.55 | 154.4±16.9 | 132.8±3.61 | 142.85±15. 5 | 139.8±2.2 |
| 11 | 127.6+26.7 | 139.33±17.1 7 | 152.97±14.6 | 127.9+3.35 | 136.01+13. 5 | 135.83±4.27 |

Experiment 2: As the Troika dataset does not contain metadata such as the height, weight and age of the subjects, another experiment was conducted inhouse, where the subjects were asked to perform the following sequence of activities in an office environment.

1. Rest: The subject had to sit on a chair in relaxed position at a given cubicle of the work environment at the 4th floor, for 1 min duration.

2. Walk: After the resting period, the subject was required to walk to the staircase of the building.

3. Climbing stairs: As soon as the subject reached the stairs, he/she had to climb the stairs as fast as he could, leading to using a lot of energy and resulting in higher heart beats. This activity was continued till the subject reached 8th floor (i.e. climbs 4 floors).

4. Walk: Once the subject reached the 8th floor, he/she had to walk through the corridor for approaching the destination.

5. Rest: At the destination the subject had to sit on a chair in a relaxed position for 1 min duration.

During the above sequence of activities, a wearable device, Samsung Gear S2™ classic Smart watch had been used for logging data from a tri-axis accelerometer sensor, and a PPG sensor. Inside the watch, a custom application had been executed for logging the data and sharing with a host machine over Wi-Fi. All the signals were sampled at 100 Hz. The cardiovascular hemodynamic model of the present disclosure was provided with the plurality of input parameters as described hereinafter.

Total blood volume: In this dataset, the height and weight of all the subjects had been noted before the experiment. Depending on this, the total blood volume, in accordance with equation (17) was calculated and tabulated as shown below.

Table 6: Total blood volume based on subject metadata

| Subject No | Height (cm) | Weight (Kg) | Age | Total Blood Volume (Liter) | BMI (kg/m$^2$) |
|---|---|---|---|---|---|
| 1 | 169 | 72 | 23 | 5.3 | 25.21 |
| 2 | 162.5 | 75 | 36 | 5.3 | 28.38 |
| 3 | 152.4 | 53 | 23 | 4.17 | 22.82 |
| 4 | 160.02 | 72 | 41 | 4.66 | 28.12 |
| 5 | 165.1 | 65 | 26 | 4.92 | 23.85 |
| 6 | 167.64 | 70 | 28 | 5.13 | 25.68 |

SVR during exercise: In this experiment, it was observed that when a subject is climbing upward on the stairs, the speed of movement varies from subject to subject making it difficult to estimate a specific MET level for the entire experiment. Therefore, an evaluation of the SVR during the exercise became difficult to estimate. To approximate the SVR, a vertical accelerometer signal was employed to estimate the MET, and then from the MET, the SVR was estimated in accordance with equation (9) above. The corresponding procedure is described below.

A collected vertical (z-axis) acceleration signal was initially passed through a low-pass filter in order to eliminate high frequency noise signals. Let us assume that the filtered vertical signal is $z_f$. Heart-rate reserve (%HRR) was calculated as

$$\%HRR = \frac{HR_{act} - HR_{rest}}{HR_{max} - HR_{rest}} \times 100 \qquad \rightarrow (18)$$

where $HR_{act}$ is the measured heart rate as obtained from the watch during the activities and $HR_{rest}$ is the mean value of the heart rate.

The maximum heart rate $HR_{max}$ was calculated as

$$HR_{max} = 220 - Age \qquad \rightarrow (19)$$

The MET level was estimated as

$$MET_{est} = a \times z_f + b \times \%HRR + c \qquad \rightarrow (20)$$

where $a$, $b$ and $c$ are constants. In the cardiovascular hemodynamic model of the present disclosure used for the experiment, the values of the constants used were $a$ = 0.0034, $b$ = 0.038, c = 3.37.

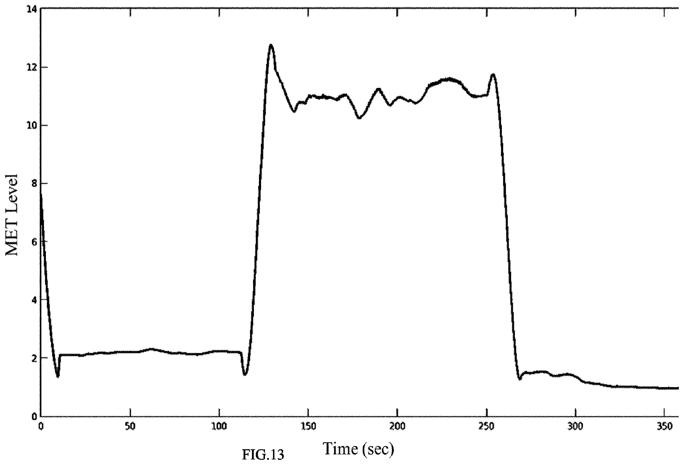

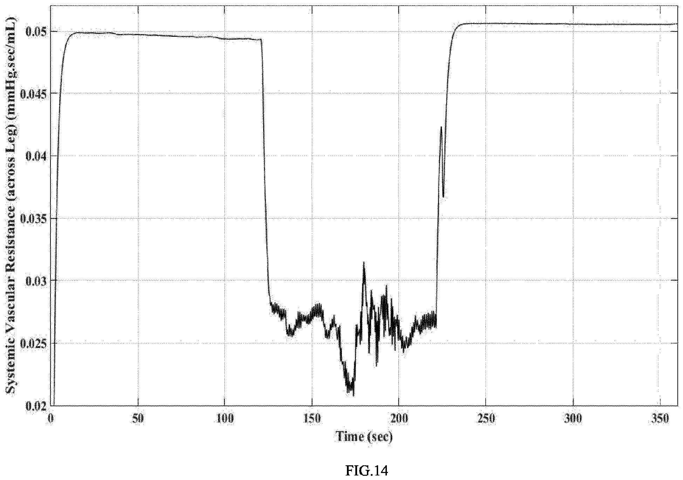

FIG. 13 illustrates the estimated MET using a vertical accelerometer signal for a subject 2, and FIG. 14 illustrates the estimated SVR using the estimated MET for the subject 2, in accordance with some embodiment of the present disclosure.

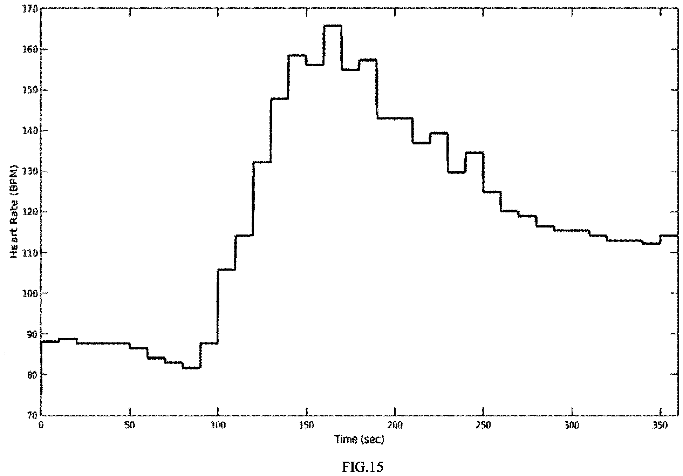

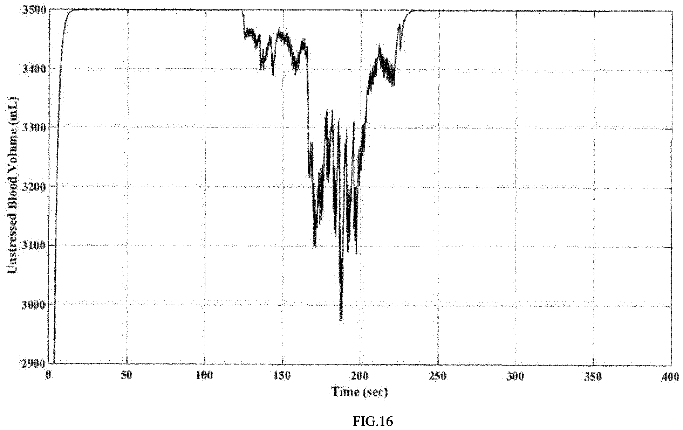

Heart rate estimation from PPG signal: During the exercise condition, the signal quality of PPG is very poor. To overcome this challenge, a time-window of 10 sec was selected and the heart rate was estimated by comparing with the accelerometer signal as described previously. FIG. 15 illustrates the estimated heart rate from PPG signal for the subject 2, in accordance with some embodiment of the present disclosure. During the simulation, the unstressed volume is approximated using the autoregulation method. FIG.16 illustrates estimated unstressed blood volume based on the autoregulation method for the subject 2, in accordance with some embodiment of the present disclosure.

By inputting the plurality of input parameters mentioned above from experiment 2 into the cardiovascular hemodynamic model of the present disclosure, the cardiac parameters such as the cardiac output, the ejection fraction and the mean arterial pressure were estimated.

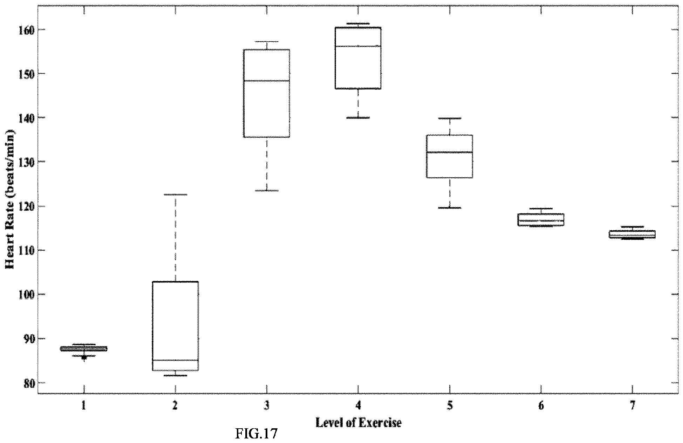

FIG. 17 illustrates estimated heart rate from PPG signal in the form of a box plot for the subject 2, in accordance with some embodiments of the present disclosure. Similar box plots were derived for all the 6 subjects considered. Table 7 below provides a comparison of the estimated heart rate at different levels of physical activities for the 6 subjects.

Table 7: Heart rate comparison

| Sub No | Levels of physical activities of TCS Dataset | | | | | | |
|---|---|---|---|---|---|---|---|
| | Level 1 (Rest) | Level 2 (Walk) | Level 3 (Walking up to (1/3)$^{rd}$ of the total stair) | Level 4 (Walking up to (2/3)$^{rd}$ of the total stair) | Level 5 (end of stair) | Level 6 (Walk) | Level 7 (Rest) |
| 1 | 89.75±3.6 | 93.76±6.15 | 118.22±5.0 7 | 131.27±5 | 126.03±2.95 | 125.4+4.63 | 112.88±3 |
| 2 | 87.57±0.74 | 93.07±12.9 | 145.04±10.86 | 153.39±7.6 | 131.14±6.34 | 116.91±1.3 | 113.57±0.9 |
| 3 | 89.85±3.34 | 92.74±1.28 | 92.32±0.2 | 108.54+11.45 | 122.32±8.37 | 118.11+5.03 | 112.71+6.35 |
| 4 | 78.19+4.16 | 84.24±6.27 | 122.01±12.3 | 138.29+11.6 | 130.97±6.6 | 113.40±2.7 | 108.18+11.34 |
| 5 | 84.91±0.82 | 94.68±11.77 | 132.98±9.5 | 126.95±10.4 | 120.85±3.32 | 114.15±2.03 | 105.74±2.16 |
| 6 | 93.44+1.35 | 94.57±2.68 | 105.35±1.8 | 133.31+18 | 139.66+14.03 | 112.83±1.24 | 113.96+1.29 |

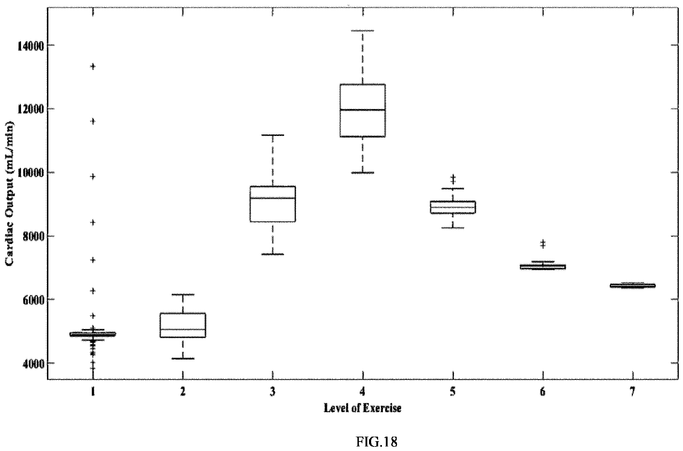

FIG. 18 illustrates estimated cardiac output in the form of a box plot for the subject 2, in accordance with some embodiments of the present disclosure. Similar box plots were derived for all the 6 subjects considered. Table 8 below provides a comparison of the estimated cardiac output at different levels of physical activities for the 6 subjects.

Table 8: Cardiac output comparison

| Subject Number | Levels of physical activities of TCS Dataset | | | | | | |
|---|---|---|---|---|---|---|---|
| | Level 1 (Rest) | Level 2 (Walk) | Level 3 (Walking up to (1/3)$^{rd}$ of the total stair) | Level 4 (Walking up to (2/3)$^{rd}$ of the total stair) | Level 5 (end of stair) | Level 6 (Walk) | Level 7 (Rest) |
| 1 | 5.6±1.2 | 6.9±1.8 | 9.6±0.55 | 12.14±0.83 | 10.88±2.6 | 8.23±0.26 | 7.25±0.2 |
| 2 | 5.04±1.25 | 5.16±0.5 | 9.17±0.95 | 12.03±0.9 | 8.89±0.3 | 7.05+0.14 | 6.42±0.05 |
| 3 | 5.13±1.51 | 5.87±2.5 | 5.85±0.7 | 8.43±1.05 | 8.65±1.5 | 5.32±0.3 | 7.01±0.4 |
| 4 | 5.37±0.9 | 6.4±0.5 | 9.82±1.01 | 13.36±1.6 | 12.27±0.86 | 8.9±0.23 | 9.01±0.95 |
| 5 | 5.14±1.4 | 6.44±0.5 | 9.46±0.88 | 11.62±1.11 | 10.78±0.33 | 8.8±0.13 | 7.6±0.16 |
| 6 | 6.1±1.48 | 8.75±1.3 | 10.97±0.47 | 14.63±1.9 | 12.81±0.95 | 9.23+0.2 | 8.8±0.1 |

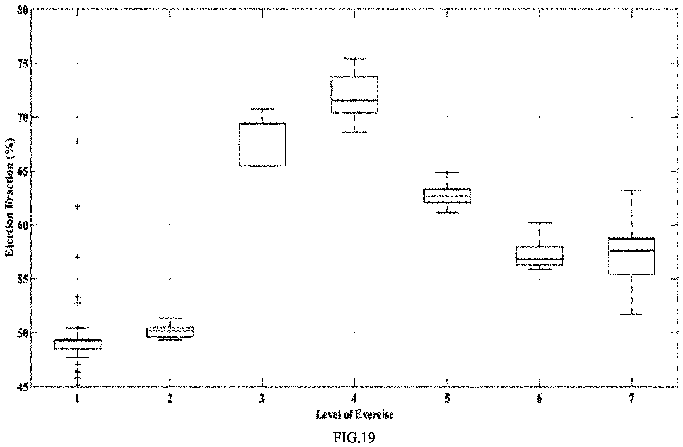

FIG. 19 illustrates estimated ejection fraction in the form of a box plot for the subject 2, in accordance with some embodiments of the present disclosure. Similar box plots were derived for all the 6 subjects considered. Table 9 below provides a comparison of the estimated ejection fraction at different levels of physical activities for the 6 subjects.

Table 9: Ejection fraction comparison

| Subject Number | Levels of physical activities of TCS Dataset | | | | | | |
|---|---|---|---|---|---|---|---|
| | Level 1 (Rest) | Level 2 (Walk) | Level 3 (Walking up to $(1/3)^{rd}$ of the total stair) | Level 4 (Walking up to $(2/3)^{rd}$ of the total stair) | Level 5 (end of stair) | Level 6 (Walk) | Level 7 (Rest) |
| 1 | 49.3±7.2 | 49.33±0.65 | 66.9±3.1 | 72.77±3.7 | 69.02±1.98 | 63.9±0.45 | 59.56±2.74 |
| 2 | 48.5±7.26 | 50.02+0.56 | 68.17+2.1 4 | 71.9+2.14 | 62.67+0.93 | 57.2±1.2 | 57.03±1.94 |
| 3 | 57.95±9.5 | 60.16±0.16 | 72.74±0.3 3 | 80.7±7.07 | 76.93±9.72 | 63.1±3.95 | 60.4±2.95 |
| 4 | 52.8±7.4 | 52.6±0.66 | 70.6+1.1 | 79.95±2.7 1 | 70.8±1.17 | 60.5±1.66 | 60.9+2.83 |
| 5 | 49.9±9.25 | 50.92±0.68 | 69.08±1.9 7 | 78.57±1.4 5 | 69.9±1.56 | 61.8±0.02 | 60.94±1.9 |
| 6 | 49.3±8.32 | 49.34±2.83 | 79.83±0.6 3 | 83.82±4.7 | 76.8±1.05 | 63.09±0.9 | 58.03±3.87 |

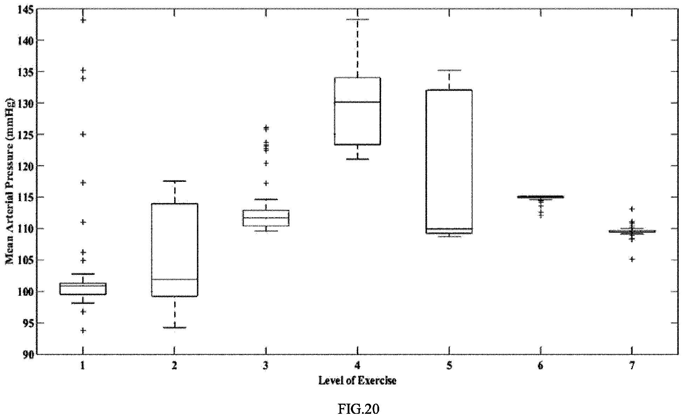

FIG.20 illustrates estimated mean arterial pressure in the form of a box plot for the subject 2, in accordance with some embodiments of the present disclosure. Similar box plots were derived for all the 6 subjects considered. Table 10 below provides a comparison of the estimated mean arterial pressure at different levels of physical activities for the 6 subjects.

Table 10: Mean arterial pressure comparison

| Subject Number | Levels of physical activities of TCS Dataset | | | | | | |
|---|---|---|---|---|---|---|---|
| | Level 1 (Rest) | Level 2 (Walk) | Level 3 (Walking up to $(1/3)^{rd}$ of the total stair) | Level 4 (Walking up to $(2/3)^{rd}$ of the total stair) | Level 5 (end of stair) | Level 6 (Walk) | Level 7 (Rest) |
| 1 | 99.8+15.3 | 118.12+21.7 | 144.75±2. 35 | 145.5±1 | 135+21.1 | 115.6±1 | 109.75±0.6 |
| 2 | 102.95t14.3 | 105.46+7.6 | 113.86±5. 25 | 129.6±5.7 5 | 117.02±10.6 | 114.8±0.6 | 109.54+0.73 |
| 3 | 86.13±19.7 | 111.4+26.5 | 111.9+12. 04 | 126.98+3. 12 | 120.04±1.7 | 98.72+2.15 | 111.95+7.36 |
| 4 | 93.23±15.02 | 105.8±1.3 | 140.1±2.2 | 157.6±6.7 | 140.9±6.9 | 125.4±4.07 | 122.4±12.35 |
| 5 | 89.25+18.2 | 98.6±1.05 | 141.4+2.8 5 | 156.5+11. 75 | 121.21±0.5 | 101.8+0.03 | 107.44±12.28 |
| 6 | 106.09±19.2 | 115.2±1.01 | 144.95+0. 8 | 181.3+11. 2 | 171.15+2.88 | 159.9±2.42 | 139.75+18.4 |

As part of a post-operative monitoring process, during therapy planning, a care giver simulates different heart rate conditions along with post-operative valvular conditions and selects different exercise levels, linked with MET value. Depending upon the selected heart rate, MET, subject metadata and underlying cardiac pathology emulated in the personalized cardiovascular hemodynamic model for the subject being monitored, cardiac parameters like cardiac output, ejection fraction and mean arterial pressure are generated by the personalized cardiovascular hemodynamic model. The simulated cardiac output provides a baseline activity response based on which, the caregiver may plan daily ambulatory activities and therapy. The method and system of the present disclosure thus overcome the challenges of the art wherein firstly cardiovascular hemodynamic models used were not personalized for the subject being monitored. Secondly, the input parameters for the personalized hemodynamic model of the present disclosure is estimated using output of wearable devices, thereby facilitating personalized cardiac rehabilitation guidance and care continuum.

The written description describes the subject matter herein to enable any person skilled in the art to make and use the embodiments. The scope of the subject matter embodiments is defined by the claims and may include other modifications that occur to those skilled in the art. Such other modifications are intended to be within the scope of the claims if they have similar elements that do not differ from the literal language of the claims or if they include equivalent elements with insubstantial differences from the literal language of the claims.

It is to be understood that the scope of the protection is extended to such a program and in addition to a computer-readable means having a message therein; such computer-readable storage means contain program-code means for implementation of one or more steps of the method, when the program runs on a server or mobile device or any suitable programmable device. The hardware device can be any kind of device which can be programmed including e.g. any kind of computer like a server or a personal computer, or the like, or any combination thereof. The device may also include means which could be e.g. hardware means like e.g. an application-specific integrated circuit (ASIC), a field-programmable gate array (FPGA), or a combination of hardware and software means, e.g. an ASIC and an FPGA, or at least one microprocessor and at least one memory with software processing components located therein. Thus, the means can include both hardware means, and software means. The method embodiments described herein could be implemented in hardware and software. The device may also include software means. Alternatively, the embodiments may be implemented on different hardware devices, e.g. using a plurality of CPUs.

The embodiments herein can comprise hardware and software elements. The embodiments that are implemented in software include but are not limited to, firmware, resident software, microcode, etc. The functions performed by various components described herein may be implemented in other components or combinations of other components. For the purposes of this description, a computer-usable or computer readable medium can be any apparatus that can comprise, store, communicate, propagate, or transport the program for use by or in connection with the instruction execution system, apparatus, or device.

The illustrated steps are set out to explain the exemplary embodiments shown, and it should be anticipated that ongoing technological development will change the manner in which particular functions are performed. These examples are presented herein for purposes of illustration, and not limitation. Further, the boundaries of the functional building blocks have been arbitrarily defined herein for the convenience of the description. Alternative boundaries can be defined so long as the specified functions and relationships thereof are appropriately performed. Alternatives (including equivalents, extensions, variations, deviations, etc., of those described herein) will be apparent to persons skilled in the relevant art(s) based on the teachings contained herein. Such alternatives fall within the scope of the disclosed embodiments. Also, the words "comprising," "having," "containing," and "including," and other similar forms are intended to be equivalent in meaning and be open ended in that an item or items following any one of these words is not meant to be an exhaustive listing of such item or items, or meant to be limited to only the listed item or items. It must also be noted that as used herein and in the appended claims, the singular forms "a," "an," and "the" include plural references unless the context clearly dictates otherwise.

Furthermore, one or more computer-readable storage media may be utilized in implementing embodiments consistent with the present disclosure. A computer-readable storage medium refers to any type of physical memory on which information or data readable by a processor may be stored. Thus, a computer-readable storage medium may store instructions for execution by one or more hardware processors, including instructions for causing the processor(s) to perform steps or stages consistent with the embodiments described herein. The term "computer-readable medium" should be understood to include tangible items and exclude carrier waves and transient signals, i.e., be non-transitory. Examples include random access memory (RAM), read-only memory (ROM), volatile memory, nonvolatile memory, hard drives, CD ROMs, DVDs, flash drives, disks, and any other known physical storage media.

It is intended that the disclosure and examples be considered as exemplary only, with a true scope of disclosed embodiments being indicated by the following claims.

## Claims

1. A processor implemented method (300) comprising the steps of:

   estimating, via one or more hardware processors, a plurality of input parameters for a personalized cardiovascular hemodynamic model associated with a subject being monitored (302),

   wherein the step of estimating a plurality of input parameters comprises sequential activation of a right atrium *ra,* a left atrium *la,* a right ventricle *rv* and a left ventricle *lv* of the personalized cardiovascular hemodynamic model using compliance functions $C_{ra}(t)$, $C_{la}(t)$, $C_{rv}(t)$ and $C_{lv}(t)$ respectively, the compliance functions being defined as:

(i) the compliance function to actuate $ra$, $C_{ra}(t) = C_{min,ra} + 0.5 \times (C_{max,ra} - C_{min,ra})u(t)$,

$$u(t) = \begin{cases} 0, & 0 \le t < T_a \\ 1 - cos\left(2\pi\frac{t-T_a}{T-T_a}\right), & T_a \le t < T \end{cases}$$

wherein , $C_{min,ra}$ and $C_{max,ra}$ are the minimum and maximum values of the $ra$ compliance, $u(t)$ is the activation function, time t is considered over a complete cardiac cycle, $T_a$ is the start of the activation of $ra$ and $T$ is the end of the cardiac cycle;

(ii) the compliance function to actuate $la$, $C_{la}(t) = C_{min,la} + 0.5 \times (c_{max,la} - C_{min,la})u(t - d_{la})$, wherein $C_{min,la}$ and $C_{max,la}$ are the minimum and maximum values of the $la$ compliance and $d_{la}$ represents a time delay between activation of the $ra$ and the $la$; and

(iii) the compliance functions to actuate $rv$ and $lv$ are represented as

$$C_i(t) = C_i \times u_v(t - d), i \in \{lv, rv\},$$

$$u_v(t) = \begin{cases} 0.5 - 0.5cos\left(\pi\frac{1}{T_1}\right), & 0 \le t < T_1 \\ 0.5 + 0.5cos\left(\pi\frac{t-T_1}{T_2-T_1}\right), & T_1 \le t < T_2 \\ 0, & T_2 \le t < T \end{cases}$$

wherein ,

$C_i$; $i \in \{lv, rv\}$ is the systolic compliance across $lv$ or $rv$ and is estimated as a ratio of R-peak and T-peak of the ECG signal, $u_v(t)$ is the activation function, d represents the time delay in activation of $lv$ or $rv$ from $ra$, and $T_1$ and $T_2$ are the systolic and diastolic activation time instances of the cardiac cycle respectively, wherein the plurality of input parameters comprising (i) Systemic Vascular Resistance (SVR), (ii) unstressed blood volume, (iii) total blood volume in a body of the subject is estimated using height and weight of the subject, and (iv) heart rate of the subject, **characterized in that** the step of

estimating comprises:

estimating the SVR using Metabolic EquivalenT (MET) levels associated with an activity level of the subject (302a), wherein the step of estimating the SVR is represented as

$$R_s(t) = \begin{cases} R_s(0) & if\ MET(t) < 2.5 \\ \frac{R_s(0)}{MET(t)} * e^{-\frac{t*MET(t)}{\tau}} & otherwise \end{cases},$$

wherein $R_s(0)$ represents the SVR at rest, MET(t) represents a metabolic equivalent of the activity performed at the $t^{th}$ time, and $\tau$ is a time constant, and wherein the SVR corresponds to a section of the body of the subject depending on the activity being performed, while the SVR of remaining sections are considered constant or modulated by the autoregulation method, the section of the body being an upper body, a middle body or a lower body of the subject; and

updating the unstressed blood volume estimated when the subject is at rest, using an autoregulation method, when an activity is performed by the subject (302b), wherein the autoregulation method comprises:

sensing aortic pressure by baroreceptors located at carotid sinus and aortic arch (302b-1);
converting the sensed aortic pressure into a neural firing frequency via afferent sympathetic pathways (302b-2);
generating sympathetic and parasympathetic nervous activities via a central nervous system and efferent pathway depending on the neural firing frequency (302b-3); and
updating an additional blood demand representing the unstressed blood volume during the activity using the generated sympathetic and parasympathetic nervous activities (302b-4); and

estimating, via the personalized cardiovascular hemodynamic model, cardiac parameters including cardiac output, ejection fraction and mean arterial pressure, using the estimated plurality of input parameters (304).

2. The processor implemented method of claim 1, wherein the step of estimating a plurality of input parameters is preceded by personalizing a cardiovascular hemodynamic model to obtain the personalized cardiovascular hemodynamic model, the personalizing being based on one or more of (i) cardiac parameters obtained from an echocardiogram, (ii) ECG signal obtained from a wearable device worn by the subject when performing the activity and (iii) metadata of the subject including height and weight associated thereof.

3. The processor implemented method of claim 1, wherein the heart rate of the subject is estimated using (i) a Photoplethysmogram (PPG) signal and an accelerometer signal or (ii) an Electrocardiogram (ECG) signal, from a wearable device worn by the subject when performing the activity.

4. A system (100) comprising:
one or more data storage devices (102) operatively coupled to one or more hardware processors (104) and configured to store instructions for execution via the one or more hardware processors to:

estimate a plurality of input parameters for a personalized cardiovascular hemodynamic model associated with a subject being monitored,

wherein the one or more processors are configured to perform sequential activation of a right atrium $ra$, a left atrium $la$, a right ventricle $rv$ and a left ventricle $lv$ of the personalized cardiovascular hemodynamic model using compliance functions $C_{ra}(t)$, $C_{la}(t)$, $C_{rv}(t)$ and $C_{lv}(t)$ respectively, the compliance functions being defined as:

(i) the compliance function to actuate $ra$, $C_{ra}(t) = C_{min,ra} + 0.5 \times (C_{max,ra} - C_{min,ra})u(t)$,

$$u(t) = \begin{cases} 0, & 0 \leq t < T_a \\ 1 - cos\left(2\pi \frac{t-T_a}{T-T_a}\right), & T_a \leq t < T \end{cases}$$

wherein , $C_{min,ra}$ and $C_{max,ra}$ are the minimum and maximum values of the $ra$ compliance, u(t) is the activation function, time t is considered over a complete cardiac cycle, $T_a$ is the start of the activation of $ra$ and $T$ is the end of the cardiac cycle;

(ii) the compliance function to actuate $la$, $C_{la}(t) = C_{min,la} + 0.5 \times (C_{max,la} - C_{min,la})u(t - d_{la})$, wherein $C_{min,la}$ and $C_{max,la}$ are the minimum and maximum values of the $la$ compliance and $d_{la}$ represents a time delay between activation of the $ra$ and the $la$; and

(iii) the compliance functions to actuate $rv$ and $lv$ are represented as

$$C_i(t) = C_i \times u_v(t - d), i \in \{lv, rv\},$$

$$u_v(t) = \begin{cases} 0.5 - 0.5cos\left(\pi \frac{1}{T_1}\right), & 0 \leq t < T_1 \\ 0.5 + 0.5cos\left(\pi \frac{t-T_1}{T_2-T_1}\right), & T_1 \leq t < T_2 \\ 0, & T_2 \leq t < T \end{cases}$$

wherein ,

$C_i; i \in \{lv, rv\}$ is the systolic compliance across $lv$ or $rv$ and is estimated as a ratio of R-peak and T-peak of the ECG signal, $u_v(t)$ is the activation function, d represents the time delay in activation of $lv$ or $rv$ from $ra$, and $T_1$ and $T_2$ are the systolic and diastolic activation time instances of the cardiac cycle respectively, wherein the plurality of input parameters comprising (i) Systemic Vascular Resistance (SVR), (ii) unstressed blood volume, (iii) total blood volume in a body of the subject is estimated using height and weight of the subject, and (iv) heart rate of the subject, **characterized in that**

estimating the plurality of input parameters comprises:

estimating the SVR using Metabolic EquivalenT (MET) levels associated with an activity level of the subject, wherein the one or more processors are configured to estimate the SVR based on

$$R_s(t) = \begin{cases} R_s(0) & if\ MET(t) < 2.5 \\ \frac{R_s(0)}{MET(t)} * e^{-\frac{t*MET(t)}{\tau}} & otherwise \end{cases},$$

wherein $R_s(0)$ represents the SVR at rest, MET(t) represents a metabolic equivalent of the activity performed at the $t^{th}$ time, and $\tau$ is a time constant, and wherein the SVR corresponds to a section of the body of the subject depending on the activity being performed, while the SVR of remaining sections are considered constant or modulated by the autoregulation method, the section of the body being an upper body, a middle body or a lower body of the subject; and
updating the unstressed blood volume estimated when the subject is at rest, using an autoregulation method, when an activity is performed by the subject, wherein the autoregulation method comprises:

sensing aortic pressure by baroreceptors located at carotid sinus and aortic arch;
converting the sensed aortic pressure into a neural firing frequency via afferent sympathetic pathways;
generating sympathetic and parasympathetic nervous activities via a central nervous system and efferent pathway depending on the neural firing frequency; and
updating an additional blood demand representing the unstressed blood volume during the activity using the generated sympathetic and parasympathetic nervous activities; and

estimate cardiac parameters including cardiac output, ejection fraction and mean arterial pressure, using the estimated plurality of input parameters.

5. The system of claim 4, wherein the one or more processors are configured to personalize a cardiovascular hemodynamic model to obtain the personalized cardiovascular hemodynamic model, prior to estimating the plurality of input parameters, based on one or more of (i) cardiac parameters obtained from an echocardiogram, (ii) ECG signal obtained from a wearable device worn by the subject when performing the activity and (iii) metadata of the subject including height and weight associated thereof.

6. The system of claim 4, wherein the one or more processors are configured to estimate the heart rate of the subject using (i) a Photoplethysmogram (PPG) signal and an accelerometer signal or (ii) an Electrocardiogram (ECG) signal, from a wearable device worn by the subject when performing the activity.

7. A computer program product comprising a non-transitory computer readable medium having a computer readable program embodied therein, wherein the computer readable program, when executed on a computing device, causes the computing device to:

estimate a plurality of input parameters for a personalized cardiovascular hemodynamic model associated with a subject being monitored,
wherein the step of estimating a plurality of input parameters comprises sequential activation of a right atrium *ra,* a left atrium *la,* a right ventricle *rv* and a left ventricle *lv* of the personalized cardiovascular hemodynamic model using compliance functions $C_{ra}(t)$, $C_{la}(t)$, $C_{rv}(t)$ and $C_{lv}(t)$ respectively, the compliance functions being defined as:

(i) the compliance function to actuate ra, $C_{ra}(t) = C_{min,ra} + 0.5 \times (C_{max,ra} - C_{min,ra})u(t)$,

$$u(t) = \begin{cases} 0, & 0 \le t < T_a \\ 1 - cos\left(2\pi \frac{t-T_a}{T-T_a}\right), & T_a \le t < T \end{cases}$$

wherein $C_{min,ra}$ and $C_{max,ra}$ are the minimum and maximum values of the *ra* compliance, $u(t)$ is the activation function, time t is considered over a complete cardiac cycle, $T_a$ is the start of the activation of *ra* and $T$ is the end of the cardiac cycle;
(ii) the compliance function to actuate *la,* $C_{la}(t) = C_{min,la} + 0.5 \times (C_{max,la} - C_{min,la})u(t - d_{la})$, wherein $C_{min,la}$ and $C_{max,la}$ are the minimum and maximum values of the *la* compliance and $d_{la}$ represents a time delay between activation of the *ra* and the *la*; and
(iii) the compliance functions to actuate *rv* and *lv* are represented as

$$C_i(t) = C_i \times u_v(t - d), i \in \{lv, rv\},$$

$$u_v(t) = \begin{cases} 0.5 - 0.5cos\left(\pi\frac{1}{T_1}\right), & 0 \leq t < T_1 \\ 0.5 + 0.5cos\left(\pi\frac{t-T_1}{T_2-T_1}\right), & T_1 \leq t < T_2 \\ 0, & T_2 \leq t < T \end{cases},$$

wherein

$C_i$; $i \in \{lv, rv\}$ is the systolic compliance across $lv$ or $rv$ and is estimated as a ratio of R-peak and T-peak of the ECG signal, $u_v(t)$ is the activation function, $d$ represents the time delay in activation of $lv$ or $rv$ from $ra$, and $T_1$ and $T_2$ are the systolic and diastolic activation time instances of the cardiac cycle respectively,

wherein the plurality of input parameters comprising (i) Systemic Vascular Resistance (SVR), (ii) unstressed blood volume, (iii) total blood volume in a body of the subject using height and weight of the subject, and (iv) heart rate of the subject using (i) a Photoplethysmogram (PPG) signal and an accelerometer signal or (ii) an Electrocardiogram (ECG) signal, from a wearable device worn by the subject when performing the activity, **characterized in that** estimating the plurality of input parameters comprises:

estimating the SVR using Metabolic EquivalenT (MET) levels associated with an activity level of the subject, wherein the step of estimating the SVR is represented as

$$R_s(t) = \begin{cases} R_s(0) & \text{if } MET(t) < 2.5 \\ \frac{R_s(0)}{MET(t)} * e^{-\frac{t*MET(t)}{\tau}} & \text{otherwise} \end{cases},$$

wherein $R_s(0)$ represents the SVR at rest, MET($t$) represents a metabolic equivalent of the activity performed at the $t^{th}$ time, and $\tau$ is a time constant, and wherein the SVR corresponds to a section of the body of the subject depending on the activity being performed, while the SVR of remaining sections are considered constant or modulated by the autoregulation method, the section of the body being an upper body, a middle body or a lower body of the subject; and
updating the unstressed blood volume estimated when the subject is at rest, using an autoregulation method, when an activity is performed by the subject, wherein the autoregulation method comprises:

sensing aortic pressure by baroreceptors located at carotid sinus and aortic arch;
converting the sensed aortic pressure into a neural firing frequency via afferent sympathetic pathways;
generating sympathetic and parasympathetic nervous activities via a central nervous system and efferent pathway depending on the neural firing frequency; and
updating an additional blood demand representing the unstressed blood volume during the activity using the generated

sympathetic and parasympathetic nervous activities; and estimate cardiac parameters including cardiac output, ejection fraction and mean arterial pressure, using the estimated plurality of input parameters.

8. The computer program product of claim 7, wherein the computer readable program further causes the computing device to personalize a cardiovascular hemodynamic model to obtain the personalized cardiovascular hemodynamic model, prior to estimating a plurality of input parameters, the personalizing being based on one or more of (i) cardiac parameters obtained from an echocardiogram, (ii) ECG signal obtained from a wearable device worn by the subject when performing the activity and (iii) metadata of the subject including height and weight associated thereof.

**Patentansprüche**

1. Prozessorimplementiertes Verfahren (300), umfassend die folgenden Schritte:

Schätzen, über einen oder mehrere Hardwareprozessoren, einer Vielzahl von Eingabeparametern für ein personalisiertes kardiovaskuläres hämodynamisches Modell, das einem überwachten Subjekt zugeordnet ist (302),

wobei der Schritt des Schätzens einer Vielzahl von Eingabeparametern sequentielle Aktivierung eines rechten Atriums $ra$, eines linken Atriums $la$, eines rechten Ventrikels $rv$ und eines linken Ventrikels $lv$ des personalisierten kardiovaskulären hämodynamischen Modells unter Verwendung von Compliance-Funktionen $C_{ra}(t)$, $C_{la}(t)$, $C_{rv}(t)$ bzw. $C_{lv}(t)$ umfasst, wobei die Compliance-Funktionen definiert sind als:

(i) die Compliance-Funktion zum Betätigen $ra$, $C_{ra}(t) = C_{min,ra} + 0.5 \times (C_{max,ra} - C_{min,ra})u(t)$,

$$u(t) = \begin{cases} 0, & 0 \leq t < T_a \\ 1 - cos\left(2\pi \frac{t-T_a}{T-T_a}\right), & T_a \leq t < T \end{cases}$$

wobei , $C_{min,ra}$ and $C_{max,ra}$ die minimalen und maximalen Werte der $ra$ Compliance sind, $u(t)$ die Aktivierungsfunktion ist, Zeit $t$ über einen vollständigen Herzzyklus betrachtet wird, $t$ der Beginn der Aktivierung von $ra$ ist und $t$ das Ende des Herzzyklus ist;

(ii) die Compliance-Funktion zum Betätigen $la$, $C_{la}(t) = C_{min,la} + 0.5 \times (C_{max,la} - C_{min,la})u(t - d_{la})$, wobei $C_{min,la}$ und $C_{max,la}$ die minimalen und maximalen Werte der $la$ Compliance sind und $d_{la}$ eine Zeitverzögerung zwischen Aktivierung des $ra$ und des $la$ darstellt; und

(iii) die Compliance-Funktionen zum Betätigen $rv$ und $lv$ dargestellt sind als

$$C_i(t) = C_i \times u_v(t - d), i \in \{lv, rv\},$$

$$u_v(t) = \begin{cases} 0.5 - 0.5cos\left(\pi \frac{1}{T_1}\right), & 0 \leq t < T_1 \\ 0.5 + 0.5cos\left(\pi \frac{t-T_1}{T_2-T_1}\right), & T_1 \leq t < T_2 \\ 0, & T_2 \leq t < T \end{cases}$$

Wobei ,

$C_i; i \in \{lv, rv\}$ die systolische Compliance über $lv$ oder $rv$ ist und als ein Verhältnis von R-Peak und T-Peak des EKG-Signals geschätzt wird, $u_v(t)$ die Aktivierungsfunktion ist, $d$ die Zeitverzögerung bei Aktivierung von $lv$ oder $rv$ von $ra$ darstellt und $T_1$ und $T_2$ die systolischen bzw. diastolischen Aktivierungszeitpunkte des Herzzyklus sind,

wobei die Vielzahl von Eingabeparametern (i) systemischen vaskulären Resistenz (SVR), (ii) unbelastetes Blutvolumen, (iii) Gesamtblutvolumen in einem Körper des Subjekts unter Verwendung von Größe und Gewicht des Subjekts und (iv) Herzfrequenz des Subjekts geschätzt wird, **dadurch gekennzeichnet, dass** der Schritt des Schätzens umfasst:

Schätzen der SVR unter Verwendung von metabolischen ÄquivalenT(MET)-Niveaus, die einem Aktivitätsniveau des Subjekts zugeordnet sind (302a), wobei der Schritt des Schätzens der SVR dargestellt ist als

$$R_s(t) = \begin{cases} R_s(0) & wenn\ MET(t) < 2.5 \\ \frac{R_s(0)}{MET(t)} * e^{-\frac{t*MET(t)}{\tau}} & sonst \end{cases}$$

wobei $R_s(0)$ die SVR im Ruhezustand darstellt, $MET(t)$ ein metabolisches Äquivalent der zum $t^{ten}$ Zeitpunkt durchgeführten Aktivität darstellt und $\tau$ eine Zeitkonstante ist, und wobei die SVR einem Abschnitt des Körpers des Subjekts in Abhängigkeit von der durchgeführten Aktivität entspricht, während die SVR der verbleibenden Abschnitte als konstant oder durch das Autoregulationsverfahren moduliert angesehen werden, wobei der Abschnitt des Körpers ein Oberkörper, ein Mittelkörper oder ein Unterkörper des Subjekts ist; und

Aktualisieren des unbelasteten Blutvolumens, das geschätzt wird, wenn sich das Subjekt im Ruhezustand befindet, unter Verwendung eines Autoregulationsverfahrens, wenn eine Aktivität durch das Subjekt durchgeführt wird (302b), wobei das Autoregulationsverfahren umfasst:

Erfassen des Aortendrucks durch Barorezeptoren, die sich an dem Karotissinus und dem Aortenbogen befinden (302b-1);

Umwandeln des erfassten Aortendrucks in eine neuronale Auslösefrequenz über afferente sympathische Pfade (302b-2);

Erzeugen von sympathischen und parasympathischen Nervenaktivitäten über ein zentrales Nervensystem und einen efferenten Pfad in Abhängigkeit von der neuronalen Auslösefrequenz (302b-3); und

Aktualisieren eines zusätzlichen Blutbedarfs, der das unbelastete Blutvolumen während der Aktivität darstellt, unter Verwendung der erzeugten sympathischen und parasympathischen Nervenaktivitäten (302b-4); und

Schätzen, über das personalisierte kardiovaskuläre hämodynamische Modell, von Herzparametern einschließlich der Herzleistung, des Auswurfanteils und des mittleren arteriellen Drucks unter Verwendung der geschätzten Vielzahl von Eingabeparametern (304).

2. Prozessorimplementiertes Verfahren nach Anspruch 1, wobei dem Schritt des Schätzens einer Vielzahl von Eingabeparametern ein Personalisieren eines kardiovaskulären hämodynamischen Modells vorausgeht, um das personalisierte kardiovaskuläre hämodynamische Modell zu erhalten, wobei das Personalisieren auf einem oder mehreren von (i) Herzparametern, die von einem Echokardiogramm erhalten werden, (ii) EKG-Signal, das von einer tragbaren Vorrichtung erhalten wird, die vom Subjekt getragen wird, wenn die Aktivität durchgeführt wird, und (iii) Metadaten des Subjekts, die damit assoziierte Größe und Gewicht umfassen.

3. Prozessorimplementiertes Verfahren nach Anspruch 1, wobei die Herzfrequenz des Subjekts unter Verwendung von (i) einem Photoplethysmogramm(PPG)-Signal und einem Beschleunigungsmessersignal oder (ii) einem Elektrokardiogramm(EKG)-Signal von einer tragbaren Vorrichtung, die vom Subjekt getragen wird, wenn die Aktivität durchgeführt wird, geschätzt wird.

4. System (100), umfassend:
eine oder mehrere Datenspeichervorrichtungen (102), die mit einem oder mehreren Hardwareprozessoren (104) wirkverbunden sind und konfiguriert sind, um Anweisungen zur Ausführung über den einen oder die mehreren Hardwareprozessoren zu speichern, um:

Schätzen einer Vielzahl von Eingabeparametern für ein personalisiertes kardiovaskuläres hämodynamisches Modell, das einem überwachten Subjekt zugeordnet ist,

wobei der eine oder die mehreren Prozessoren konfiguriert sind, um sequentielle Aktivierung eines rechten Atriums $ra$, eines linken Atriums $la$, eines rechten Ventrikels $rv$ und eines linken Ventrikels $lv$ des personalisierten kardiovaskulären hämodynamischen Modells unter Verwendung von Compliance-Funktionen $C_{ra}(t)$, $C_{la}(t)$, $C_{rv}(t)$ bzw. $C_{lv}(t)$ durchzuführen, wobei die Compliance-Funktionen definiert sind als:

(i) die Compliance-Funktion zum Betätigen $ra$, $C_{ra}(t) = C_{min,ra} + 0.5 \times (C_{max,ra} - C_{min,ra})u(t)$,

$$u(t) = \begin{cases} 0, & 0 \leq t < T_a \\ 1 - cos\left(2\pi \frac{t-T_a}{T-T_a}\right), & T_a \leq t < T \end{cases}$$

wobei , $C_{min,ra}$ und $C_{max,ra}$ die minimalen und maximalen Werte der $ra$ Compliance sind, u(t) die Aktivierungsfunktion ist, Zeit $t$ über einen vollständigen Herzzyklus betrachtet wird, $T_a$ der Beginn der Aktivierung von $ra$ ist und $T$ das Ende des Herzzyklus ist;

(ii) die Compliance-Funktion zum Betätigen $la$, $C_{la}(t) = C_{min,la} + 0.5 \times (C_{max,la} - C_{min,la})u(t - d_{la})$, wobei $C_{min,la}$ und $C_{max,la}$ die minimalen und maximalen Werte der $la$ Compliance sind und $d_{la}$ eine Zeitverzögerung zwischen Aktivierung des $ra$ und des $la$ darstellt; und

(iii) die Compliance-Funktionen zum Betätigen $rv$ und $lv$ dargestellt sind als

$$C_i(t) = C_i \times u_v(t - d), i \in \{lv, rv\},$$

$$u_v(t) = \begin{cases} 0.5 - 0.5cos\left(\pi\frac{1}{T_1}\right), & 0 \leq t < T_1 \\ 0.5 + 0.5cos\left(\pi\frac{t-T_1}{T_2-T_1}\right), & \begin{array}{l} T_1 \leq t < T_2 \\ T_2 \leq t < T \end{array} \\ 0, & \end{cases}$$

wobei

$C_i$; $i \in \{lv, rv\}$ die systolische Compliance über *lv* oder *rv* ist und als ein Verhältnis von R-Peak und T-Peak des EKG-Signals geschätzt wird, $u_v(t)$ die Aktivierungsfunktion ist, d die Zeitverzögerung bei Aktivierung von *lv* oder *rv* von *ra* darstellt und $T_1$ und $T_2$ die systolischen bzw. diastolischen Aktivierungszeitpunkte des Herzzyklus sind, wobei die Vielzahl von Eingabeparametern (i) systemischen vaskulären Resistenz (SVR), (ii) unbelastetes Blutvolumen, (iii) Gesamtblutvolumen in einem Körper des Subjekts unter Verwendung von Größe und Gewicht des Subjekts und (iv) Herzfrequenz des Subjekts geschätzt wird, **dadurch gekennzeichnet, dass** das Schätzen der Vielzahl von Eingabeparametern umfasst:

Schätzen der SVR unter Verwendung von metabolischen ÄquivalenT(MET)-Niveaus, die einem Aktivitätsniveau des Subjekts zugeordnet sind, wobei der eine oder die mehreren Prozessoren konfiguriert sind, um die SVR basierend auf

$$R_s(t) = \begin{cases} R_s(0) & wenn\ MET(t) < 2.5 \\ \frac{R_s(0)}{MET(t)} * e^{-\frac{t*MET(t)}{\tau}} & sonst \end{cases},$$

wobei $R_s(0)$ die SVR im Ruhezustand darstellt, $MET(t)$ ein metabolisches Äquivalent der zum *t*-ten Zeitpunkt durchgeführten Aktivität darstellt und $\tau$ eine Zeitkonstante ist, und wobei die SVR einem Abschnitt des Körpers des Subjekts in Abhängigkeit von der durchgeführten Aktivität entspricht, während die SVR der verbleibenden Abschnitte als konstant oder durch das Autoregulationsverfahren moduliert angesehen werden, wobei der Abschnitt des Körpers ein Oberkörper, ein Mittelkörper oder ein Unterkörper des Subjekts ist; und

Aktualisieren des unbelasteten Blutvolumens, das geschätzt wird, wenn sich das Subjekt im Ruhezustand befindet, unter Verwendung eines Autoregulationsverfahrens, wenn eine Aktivität durch das Subjekt durchgeführt wird, wobei das Autoregulationsverfahren umfasst:

Erfassen des Aortendrucks durch Barorezeptoren, die sich an dem Karotissinus und dem Aortenbogen befinden;
Umwandeln des erfassten Aortendrucks in eine neuronale Auslösefrequenz über afferente sympathische Pfade;
Erzeugen von sympathischen und parasympathischen Nervenaktivitäten über ein zentrales Nervensystem und einen efferenten Pfad in Abhängigkeit von der neuronalen Auslösefrequenz; und
Aktualisieren eines zusätzlichen Blutbedarfs, der das unbelastete Blutvolumen während der Aktivität darstellt, unter Verwendung der erzeugten sympathischen und parasympathischen Nervenaktivitäten; und

Schätzen von Herzparametern einschließlich der Herzleistung, des Auswurfanteils und des mittleren arteriellen Drucks unter Verwendung der geschätzten Vielzahl von Eingabeparametern.

5. System nach Anspruch 4, wobei der eine oder die mehreren Prozessoren konfiguriert sind, um ein kardiovaskuläres hämodynamisches Modell zu personalisieren, um das personalisierte kardiovaskuläre hämodynamische Modell zu erhalten, bevor die Vielzahl von Eingabeparametern geschätzt wird, basierend auf einem oder mehreren von (i) Herzparametern, die von einem Echokardiogramm erhalten werden, (ii) EKG-Signal, das von einer tragbaren Vorrichtung erhalten wird, die vom Subjekt getragen wird, wenn die Aktivität durchgeführt wird, und (iii) Metadaten des Subjekts, die damit assoziierte Größe und Gewicht umfassen.

6. System nach Anspruch 4, wobei der eine oder die mehreren Prozessoren konfiguriert sind, um die Herzfrequenz des Subjekts unter Verwendung von (i) einem Photoplethysmogramm(PPG)-Signal und einem Beschleunigungsmessersignal oder (ii) einem Elektrokardiogramm(EKG)-Signal von einer tragbaren Vorrichtung, die vom Subjekt getragen wird, wenn die Aktivität durchgeführt wird, zu schätzen.

7. Computerprogrammprodukt, umfassend ein nichtflüchtiges computerlesbares Medium mit einem darin verkörperten computerlesbaren Programm, wobei das computerlesbare Programm, wenn es auf einer Rechenvorrichtung ausgeführt wird, die Rechenvorrichtung zu Folgendem veranlasst:

Schätzen einer Vielzahl von Eingabeparametern für ein personalisiertes kardiovaskuläres hämodynamisches Modell, das einem überwachten Subjekt zugeordnet ist,

wobei der Schritt des Schätzens einer Vielzahl von Eingabeparametern sequentielle Aktivierung eines rechten Atriums *ra*, eines linken Atriums *la*, eines rechten Ventrikels *rv* und eines linken Ventrikels *lv* des personalisierten kardiovaskulären hämodynamischen Modells unter Verwendung von Compliance-Funktionen $C_{ra}(t)$, $C_{la}(t)$, $C_{rv}(t)$ bzw. $C_{lv}(t)$ umfasst, wobei die Compliance-Funktionen definiert sind als:

(i) die Compliance-Funktion zum Betätigen *ra*, $C_{ra}(t) = C_{min,ra} + 0.5 (C_{max,ra} C_{min,ra})u(t)$,

$$u(t) = \begin{cases} 0, & 0 \leq t < T_a \\ 1 - cos\left(2\pi\frac{t-T_a}{T-T_a}\right), & T_a \leq t < T \end{cases}$$

wobei $C_{min,ra}$ und $C_{max,ra}$ die minimalen und maximalen Werte der *ra* Compliance sind, $u(t)$ die Aktivierungsfunktion ist, Zeit *t* über einen vollständigen Herzzyklus betrachtet wird, $T_a$ der Beginn der Aktivierung von *ra* ist und *T* das Ende des Herzzyklus ist;

(ii) die Compliance-Funktion zum Betätigen *la*, $C_{la}(t) = C_{min,la} + 0.5 \times (C_{max,la} - C_{min,la})u(t - d_{la})$, wobei $C_{min,la}$ und $C_{max,la}$ die minimalen und maximalen Werte der *la* Compliance sind und $d_{la}$ eine Zeitverzögerung zwischen Aktivierung des *ra* und des la darstellt; und

(iii) die Compliance-Funktionen zum Betätigen *rv* und *lv* dargestellt sind als

$$C_i(t) = C_i \times u_v(t - d), i \in \{lv, rv\},$$

$$u_v(t) = \begin{cases} 0.5 - 0.5cos\left(\pi\frac{1}{T_1}\right), & 0 \leq t < T_1 \\ 0.5 + 0.5cos\left(\pi\frac{t-T_1}{T_2-T_1}\right), & T_1 \leq t < T_2 \\ 0, & T_2 \leq t < T \end{cases},$$

wobei $C_i; i \in \{lv, rv\}$ die systolische Compliance über *lv* oder *rv* ist und als ein Verhältnis von R-Peak und T-Peak des EKG-Signals geschätzt wird, $u_v(t)$ die Aktivierungsfunktion ist, *d* die Zeitverzögerung bei Aktivierung von *lv* oder *rv* von *ra* darstellt und $T_1$ und $T_2$ die systolischen bzw. diastolischen Aktivierungszeitpunkte des Herzzyklus sind,

wobei die Vielzahl von Eingabeparametern (i) systemischen vaskulären Resistenz (SVR), (ii) unbelastetes Blutvolumen, (iii) Gesamtblutvolumen in einem Körper des Subjekts unter Verwendung von Größe und Gewicht des Subjekts und (iv) Herzfrequenz des Subjekts unter Verwendung von (i) einem Photoplethysmogramm(PPG)-Signal und einem Beschleunigungsmessersignal oder (ii) einem Elektrokardiogramm(EKG)-Signal von einer tragbaren Vorrichtung, die vom Subjekt getragen wird, wenn die Aktivität durchgeführt wird, umfasst, **dadurch gekennzeichnet, dass** das Schätzen der Vielzahl von Eingabeparametern umfasst:

Schätzen der SVR unter Verwendung von metabolischen ÄquivalenT(MET)-Niveaus, die einem Aktivitätsniveau des Subjekts zugeordnet sind, wobei der Schritt des Schätzens der SVR dargestellt ist als

$$R_s(t) = \begin{cases} R_s(0) & wenn\ MET(t) < 2.5 \\ \frac{R_s(0)}{MET(t)} * e^{-\frac{t*MET(t)}{\tau}} & sonst \end{cases},$$

wobei $R_s(0)$ die SVR im Ruhezustand darstellt, *MET(t)* ein metabolisches Äquivalent der zum *t*-ten Zeitpunkt durchgeführten Aktivität darstellt und $\tau$ eine Zeitkonstante ist, und wobei die SVR einem Abschnitt des Körpers des Subjekts in Abhängigkeit von der durchgeführten Aktivität entspricht, während die SVR der verbleibenden Abschnitte als konstant oder durch das Autoregulationsverfahren moduliert angesehen werden, wobei der Abschnitt des Körpers ein Oberkörper, ein Mittelkörper oder ein Unterkörper des Subjekts ist; und

Aktualisieren des unbelasteten Blutvolumens, das geschätzt wird, wenn sich das Subjekt im Ruhezustand befindet, unter Verwendung eines Autoregulationsverfahrens, wenn eine Aktivität durch das Subjekt durchgeführt wird, wobei das Autoregulationsverfahren umfasst:

Erfassen des Aortendrucks durch Barorezeptoren, die sich an dem Karotissinus und dem Aortenbogen befinden;

Umwandeln des erfassten Aortendrucks in eine neuronale Auslösefrequenz über afferente sympathische Pfade;

Erzeugen von sympathischen und parasympathischen Nervenaktivitäten über ein zentrales Nervensystem und einen efferenten Pfad in Abhängigkeit von der neuronalen Auslösefrequenz; und

Aktualisieren eines zusätzlichen Blutbedarfs, der das unbelastete Blutvolumen während der Aktivität darstellt, unter Verwendung der erzeugten sympathischen und parasympathischen Nervenaktivitäten; und

Schätzen von Herzparametern einschließlich der Herzleistung, des Auswurfanteils und des mittleren arteriellen Drucks unter Verwendung der geschätzten Vielzahl von Eingabeparametern.

8. Computerprogrammprodukt nach Anspruch 7, wobei das computerlesbare Programm ferner bewirkt, dass die Rechenvorrichtung ein kardiovaskuläres hämodynamisches Modell personalisiert, um das personalisierte kardiovaskuläre hämodynamische Modell zu erhalten, bevor eine Vielzahl von Eingabeparametern geschätzt wird, wobei das Personalisieren auf einem oder mehreren von (i) Herzparametern, die von einem Echokardiogramm erhalten werden, (ii) EKG-Signal, das von einer tragbaren Vorrichtung erhalten wird, die vom Subjekt getragen wird, wenn die Aktivität durchgeführt wird, und (iii) Metadaten des Subjekts, die damit assoziierte Größe und Gewicht umfassen.

## Revendications

1. Procédé mis en oeuvre par processeur (300), comprenant les étapes ci-dessous consistant à :
estimer, par l'intermédiaire d'un ou plusieurs processeurs matériels, une pluralité de paramètres d'entrée pour un modèle d'hémodynamique cardiovasculaire personnalisé associé à un sujet surveillé (302),

dans laquelle l'étape d'estimation d'une pluralité de paramètres d'entrée comprend une activation séquentielle d'un atrium droit $ra$, d'un atrium gauche $la$, d'un ventricule droit $rv$ et d'un ventricule gauche $lv$ du modèle d'hémodynamique cardiovasculaire personnalisé au moyen de fonctions de conformité $C_{ra}(t)$, $C_{la}(t)$, $C_{rv}(t)$ et $C_{lv}(t)$ respectivement, les fonctions de conformité étant définies comme suit :

(i) la fonction de conformité pour actionner ra, $C_{ra}(t) = C_{min,ra} + 0,5 \times (C_{max,ra} - C_{min,ra})u(t)$,

$$u(t) = \begin{cases} 0, & 0 \leq t < T_a \\ 1 - cos\left(2\pi \frac{t-T_a}{T-T_a}\right), & T_a \leq t < T \end{cases}$$

dans laquelle , « $C_{min,ra}$ » et « $C_{max,ra}$ » sont les valeurs minimale et maximale de la conformité de ra, « $u(t)$ » est la fonction d'activation, le temps « $t$ » est considéré sur un cycle cardiaque complet, « $T_a$ » est le début de l'activation de ra et « $T$ » est la fin du cycle cardiaque ;

(ii) la fonction de conformité pour actionner $la$, $C_{la}(t) = C_{min,la} + 0,5 \times (C_{max,la} - C_{min,la})u(t) - d_{la})$, dans laquelle « $C_{min,la}$ » et « $C_{max,la}$ » sont les valeurs minimale et maximale de la conformité de $la$, et « $d_{la}$ » représente un délai temporel entre l'activation de ra et de la ; et

(iii) les fonctions de conformité pour actionner $rv$ et $lv$ sont représentées comme suit :

$$C_i(t) = C_i \times u_v(t - d), i \in \{lv, rv\},$$

$$u_v(t) = \begin{cases} 0,5 - 0,5cos\left(\pi\frac{1}{T_1}\right), & 0 \leq t < T_1 \\ 0,5 + 0,5cos\left(\pi\frac{t-T_1}{T_2-T_1}\right), & T_1 \leq t < T_2, \\ & T_2 \leq t < T \\ 0, & \end{cases}$$

dans laquelle

$C_i$, $i \in \{lv, rv\}$ est la conformité systolique à travers $lv$ ou $rv$, et est estimée en tant qu'un rapport entre un pic R et un pic T du signal ECG, « $u_v(t)$ » est la fonction d'activation, « $d$ » représente le délai temporel dans le cadre de l'activation de $lv$ ou $rv$ à partir de ra, et « $T_1$ » et « $T_2$ » sont les instances temporelles d'activation systolique et diastolique du cycle cardiaque, respectivement,

dans lequel la pluralité de paramètres d'entrée comprend (i) une résistance vasculaire systémique (SVR), (ii) un volume sanguin non sollicité, (iii) un volume sanguin total dans un corps du sujet qui est estimé en faisant appel à la taille et au poids du sujet, et (iv) la fréquence cardiaque du sujet, **caractérisé en ce que** l'étape d'estimation comprend les étapes ci-dessous consistant à :

estimer la résistance SVR en faisant appel à des niveaux d'équivalent métabolique (MET) associés à un niveau d'activité du sujet (302a), dans laquelle l'étape d'estimation de la résistance SVR est représentée comme suit :

$$R_s(t) = \begin{cases} R_s(0) & si \quad MET(t) < 2.5 \\ \frac{R_s(0)}{MET(t)} * e^{-\frac{t*MET(t)}{\tau}} & sinon \end{cases} ;$$

dans lequel $R_s(0)$ représente la résistance SVR au repos, « $MET(t)$ » représente un équivalent métabolique de l'activité mise en oeuvre à l'instant $t^{th}$, et « $\tau$ » est une constante de temps, et dans lequel la résistance SVR correspond à une section du corps du sujet en fonction de l'activité mise en oeuvre, tandis que la résistance SVR de sections restantes est considérée comme constante ou modulée par le procédé d'autorégulation, la section du corps étant une partie supérieure du corps, une partie intermédiaire du corps ou une partie inférieure du corps du sujet ; et

mettre à jour le volume sanguin non sollicité estimé lorsque le sujet est au repos, en faisant appel à un procédé d'autorégulation, lorsqu'une activité est mise en oeuvre par le sujet (302b), dans lequel le procédé d'autorégulation comprend les étapes ci-dessous consistant à :

détecter une pression aortique par le biais de barorécepteurs situés au niveau du sinus carotidien et de l'arc aortique (302b-1) ;
convertir la pression aortique détectée en une fréquence de décharge neuronale par l'intermédiaire de voies sympathiques afférentes (302b-2) ;
générer des activités nerveuses sympathiques et parasympathiques par l'intermédiaire d'un système nerveux central et d'une voie efférente en fonction de la fréquence de décharge neuronale (302b-3) ; et
mettre à jour une demande de sang supplémentaire représentant le volume sanguin non sollicité pendant l'activité, en faisant appel à des activités nerveuses sympathiques et parasympathiques générées (302b-4) ; et

estimer, par l'intermédiaire du modèle d'hémodynamique cardiovasculaire personnalisé, des paramètres cardiaques incluant un débit cardiaque, une fraction d'éjection et une pression artérielle moyenne, en faisant appel à la pluralité estimée de paramètres d'entrée (304).

2. Procédé mis en oeuvre par processeur selon la revendication 1, dans lequel l'étape d'estimation d'une pluralité de paramètres d'entrée est précédée par une étape de personnalisation d'un modèle d'hémodynamique cardiovasculaire en vue d'obtenir le modèle d'hémodynamique cardiovasculaire personnalisé, l'étape de personnalisation étant basée sur un ou plusieurs des éléments parmi (i) des paramètres cardiaques obtenus à partir d'un échocardiogramme, (ii) un signal ECG obtenu à partir d'un dispositif vestimentaire porté par le sujet lors de la mise en oeuvre de l'activité, et (iii) des métadonnées du sujet, y compris la taille et le poids qui lui sont associés.

3. Procédé mis en oeuvre par processeur selon la revendication 1, dans lequel la fréquence cardiaque du sujet est estimée en faisant appel à (i) un signal de photopléthysmogramme (PPG) et un signal d'accéléromètre, ou (ii) un signal d'électrocardiogramme (ECG), à partir d'un dispositif vestimentaire porté par le sujet lors de la mise en oeuvre de l'activité.

4. Système (100) comprenant :
un ou plusieurs dispositifs de stockage de données (102) couplés de manière opérationnelle à un ou plusieurs processeurs matériels (104), et configurés de manière à stocker des instructions destinées à être exécutées par ledit un ou lesdits plusieurs processeurs matériels en vue de mettre en oeuvre les étapes ci-dessous consistant à :
estimer une pluralité de paramètres d'entrée pour un modèle d'hémodynamique cardiovasculaire personnalisé associé à un sujet surveillé,

dans lesquels ledit un ou lesdits plusieurs processeurs sont configurés de manière à mettre en oeuvre une activation séquentielle d'un atrium droit *ra,* d'un atrium gauche *la,* d'un ventricule droit *rv* et d'un ventricule gauche *lv* du modèle d'hémodynamique cardiovasculaire personnalisé au moyen de fonctions de conformité $C_{ra}(t)$, $C_{la}(t)$, $C_{rv}(t)$ et $C_{lv}(t)$ respectivement, les fonctions de conformité étant définies comme suit :

(i) la fonction de conformité pour actionner ra, $C_{ra}(t) = C_{min,ra} + 0{,}5 \times (C_{max,ra} - C_{min,ra})u(t)$,

dans laquelle
$$u(t) = \begin{cases} 0, & 0 \le t < T_a \\ 1 - cos\left(2\pi\frac{t-T_a}{T-T_a}\right), & T_a \le t < T \end{cases}$$
, « $C_{min,ra}$ » et « $C_{max,ra}$ » sont les valeurs minimale et maximale de la conformité de *ra,* « $u(t)$ » est la fonction d'activation, le temps « $t$ » est considéré sur un cycle cardiaque complet, « $T_a$ » est le début de l'activation de *ra* et « $T$ » est la fin du cycle cardiaque ;

(ii) la fonction de conformité pour actionner *la,* $C_{la}(t) = C_{min,la} + 0{,}5 \times (C_{max,la} - C_{min,la})u(t) - d_{la})$, dans laquelle « $C_{min,la}$ » et « $C_{max,la}$ » sont les valeurs minimale et maximale de la conformité de *la,* et « $d_{la}$ » représente un délai temporel entre l'activation de *ra* et de *la* ; et

(iii) les fonctions de conformité pour actionner *rv* et *lv* sont représentées comme suit :

$$C_i(t) = C_i \times u_v(t - d), i \in \{lv, rv\},$$

dans laquelle
$$u_v(t) = \begin{cases} 0.5 - 0.5cos\left(\pi\frac{1}{T_1}\right), & 0 \le t < T_1 \\ 0.5 + 0.5cos\left(\pi\frac{t-T_1}{T_2-T_1}\right), & T_1 \le t < T_2 \\ 0, & T_2 \le t < T \end{cases}$$
,

$C_i$, $i \in \{lv, rv\}$ est la conformité systolique à travers *lv* ou *rv,* et est estimée en tant qu'un rapport entre un pic R et un pic T du signal ECG, « $u_v(t)$ » est la fonction d'activation, « $d$ » représente le délai temporel dans le cadre de l'activation de *lv* ou *rv* à partir de *ra,* et « $T_1$ » et « $T_2$ » sont les instances temporelles d'activation systolique et diastolique du cycle cardiaque, respectivement,

dans lequel la pluralité de paramètres d'entrée comprend (i) une résistance vasculaire systémique (SVR), (ii) un volume sanguin non sollicité, (iii) un volume sanguin total dans un corps du sujet qui est estimé en faisant appel à la taille et au poids du sujet, et (iv) la fréquence cardiaque du sujet, **caractérisé en ce que** l'étape d'estimation comprend les étapes ci-dessous consistant à :

estimer la résistance SVR en faisant appel à des niveaux d'équivalent métabolique (MET) associés à un niveau d'activité du sujet, dans lesquels ledit un ou lesdits plusieurs processeurs sor *si* figurés de manière à estimer la résistance SVR sur la base de :

$$R_s(t) = \begin{cases} R_s(0) & if\ MET(t) < 2.5 \\ \frac{R_s(0)}{MET(t)} * e^{-\frac{t*MET(t)}{\tau}} & sinon \end{cases};$$

dans lequel $R_s(0)$ représente la résistance SVR au repos, « $MET(t)$ » représente un équivalent métabolique de l'activité mise en oeuvre à l'instant $t^{th}$, et « $\tau$ » est une constante de temps, et dans lequel la résistance SVR correspond à une section du corps du sujet en fonction de l'activité mise en oeuvre, tandis que la résistance SVR de sections restantes est considérée comme constante ou modulée par le procédé d'autorégulation, la section du corps étant une partie supérieure du corps, une partie intermédiaire du corps ou une partie inférieure du corps du sujet ; et

mettre à jour le volume sanguin non sollicité estimé lorsque le sujet est au repos, en faisant appel à un procédé d'autorégulation, lorsqu'une activité est mise en oeuvre par le sujet, dans lequel le procédé d'autorégulation comprend les étapes ci-dessous consistant à :

détecter une pression aortique par le biais de barorécepteurs situés au niveau du sinus carotidien et de l'arc aortique ;
convertir la pression aortique détectée en une fréquence de décharge neuronale par l'intermédiaire de voies sympathiques afférentes ;

générer des activités nerveuses sympathiques et parasympathiques par l'intermédiaire d'un système nerveux central et d'une voie efférente en fonction de la fréquence de décharge neuronale ; et mettre à jour une demande de sang supplémentaire représentant le volume sanguin non sollicité pendant l'activité, en faisant appel à des activités nerveuses sympathiques et parasympathiques générées ; et

estimer des paramètres cardiaques incluant un débit cardiaque, une fraction d'éjection et une pression artérielle moyenne, en faisant appel à la pluralité estimée de paramètres d'entrée.

**5.** Système selon la revendication 4, dans lequel ledit un ou lesdits plusieurs processeurs sont configurés de manière à personnaliser un modèle d'hémodynamique cardiovasculaire en vue d'obtenir le modèle d'hémodynamique cardiovasculaire personnalisé, avant d'estimer la pluralité de paramètres d'entrée, sur la base d'un ou plusieurs des éléments parmi (i) des paramètres cardiaques obtenus à partir d'un échocardiogramme, (ii) un signal ECG obtenu à partir d'un dispositif vestimentaire porté par le sujet lors de la mise en oeuvre de l'activité, et (iii) des métadonnées du sujet, y compris la taille et le poids qui lui sont associés.

**6.** Système selon la revendication 4, dans lequel ledit un ou lesdits plusieurs processeurs sont configurés de manière à estimer la fréquence cardiaque du sujet en faisant appel à (i) un signal de photopléthysmogramme (PPG) et un signal d'accéléromètre, ou (ii) un signal d'électrocardiogramme (ECG), à partir d'un dispositif vestimentaire porté par le sujet lors de la mise en oeuvre de l'activité.

**7.** Produit-programme informatique comprenant un support non transitoire lisible par ordinateur dans lequel est incorporé un programme lisible par ordinateur, dans lequel le programme lisible par ordinateur, lorsqu'il est exécuté sur un dispositif informatique, amène le dispositif informatique à mettre en oeuvre les étapes ci-dessous consistant à:

estimer une pluralité de paramètres d'entrée pour un modèle d'hémodynamique cardiovasculaire personnalisé associé à un sujet surveillé ;

dans laquelle l'étape d'estimation d'une pluralité de paramètres d'entrée comprend une activation séquentielle d'un atrium droit $ra$, d'un atrium gauche $la$, d'un ventricule droit $rv$ et d'un ventricule gauche $lv$ du modèle d'hémodynamique cardiovasculaire personnalisé au moyen de fonctions de conformité $C_{ra}(t)$, $C_{la}(t)$, $C_{rv}(t)$ et $C_{lv}(t)$ respectivement, les fonctions de conformité étant définies comme suit :

(i) la fonction de conformité pour actionner ra, $C_{ra}(t) = C_{min,ra} + 0{,}5 \times (C_{max,ra} - C_{min,ra})u(t)$,

$$u(t) = \begin{cases} 0, & 0 \le t < T_a \\ 1 - cos\left(2\pi \frac{t-T_a}{T-T_a}\right), & T_a \le t < T \end{cases}$$

dans laquelle , « $C_{min,ra}$ » et « $C_{max,ra}$ » sont les valeurs minimale et maximale de la conformité de $ra$, « $u(t)$ » est la fonction d'activation, le temps « $t$ » est considéré sur un cycle cardiaque complet, « $T_a$ » est le début de l'activation de ra et « $T$ » est la fin du cycle cardiaque ;

(ii) la fonction de conformité pour actionner la, $C_{la}(t) = C_{min,la} + 0{,}5 \times (C_{max,la} - C_{min,la})u(t) - d_{la})$, dans laquelle « $C_{min,la}$ » et « $C_{max,la}$ » sont les valeurs minimale et maximale de la conformité de $la$, et « $d_{la}$ » représente un délai temporel entre l'activation de $ra$ et de $la$ ; et

(iii) les fonctions de conformité pour actionner $rv$ et $lv$ sont représentées comme suit :

$$C_i(t) = C_i \times u_v(t - d), i \in \{lv, rv\},$$

$$u_v(t) = \begin{cases} 0.5 - 0.5cos\left(\pi \frac{1}{T_1}\right), & 0 \le t < T_1 \\ 0.5 + 0.5cos\left(\pi \frac{t-T_1}{T_2 - T_1}\right), & T_1 \le t < T_2 \\ 0, & T_2 \le t < T \end{cases}$$

dans laquelle ,

$C_i$, $i \in \{lv, rv\}$ est la conformité systolique à travers $lv$ ou $rv$, et est estimée en tant qu'un rapport entre un pic R et un pic T du signal ECG, « $u_v(t)$ » est la fonction d'activation, « $d$ » représente le délai temporel dans le cadre de l'activation de $lv$ ou $rv$ à partir de $ra$, et « $T_1$ » et « $T_2$ » sont les instances temporelles d'activation systolique et diastolique du cycle cardiaque, respectivement ;

dans lequel la pluralité de paramètres d'entrée comprend (i) une résistance vasculaire systémique (SVR),

## EP 4 060 675 B1

(ii) un volume sanguin non sollicité, (iii) un volume sanguin total dans un corps du sujet qui est estimé en faisant appel à la taille et au poids du sujet, et (iv) la fréquence cardiaque du sujet, **caractérisé en ce que** l'étape d'estimation comprend les étapes ci-dessous consistant à :

estimer la résistance SVR en faisant appel à des niveaux d'équivalent métabolique (MET) associés à un niveau d'activité du sujet, dans laquelle l'étape d'estimation de la résistance SVR est représentée comme suit :

$$R_s(t) = \begin{cases} R_s(0) & si \quad MET(t) < 2.5 \\ \frac{R_s(0)}{MET(t)} * e^{-\frac{t*MET(t)}{\tau}} & sinon \end{cases} ;$$

dans lequel $R_s(0)$ représente la résistance SVR au repos, « $MET(t)$ » représente un équivalent métabolique de l'activité mise en oeuvre à l'instant $t$th, et « $\tau$ » est une constante de temps, et dans lequel la résistance SVR correspond à une section du corps du sujet en fonction de l'activité mise en oeuvre, tandis que la résistance SVR de sections restantes est considérée comme constante ou modulée par le procédé d'autorégulation, la section du corps étant une partie supérieure du corps, une partie intermédiaire du corps ou une partie inférieure du corps du sujet ; et

mettre à jour le volume sanguin non sollicité estimé lorsque le sujet est au repos, en faisant appel à un procédé d'autorégulation, lorsqu'une activité est mise en oeuvre par le sujet, dans lequel le procédé d'autorégulation comprend les étapes ci-dessous consistant à :

détecter une pression aortique par le biais de barorécepteurs situés au niveau du sinus carotidien et de l'arc aortique ;
convertir la pression aortique détectée en une fréquence de décharge neuronale par l'intermédiaire de voies sympathiques afférentes ;
générer des activités nerveuses sympathiques et parasympathiques par l'intermédiaire d'un système nerveux central et d'une voie efférente en fonction de la fréquence de décharge neuronale ; et
mettre à jour une demande de sang supplémentaire représentant le volume sanguin non sollicité pendant l'activité, en faisant appel à des activités nerveuses sympathiques et parasympathiques générées ; et

estimer des paramètres cardiaques incluant un débit cardiaque, une fraction d'éjection et une pression artérielle moyenne, en faisant appel à la pluralité estimée de paramètres d'entrée.

8. Produit-programme informatique selon la revendication 7, dans lequel le programme lisible par ordinateur amène en outre le dispositif informatique à personnaliser un modèle d'hémodynamique cardiovasculaire en vue d'obtenir le modèle d'hémodynamique cardiovasculaire personnalisé, avant d'estimer une pluralité de paramètres d'entrée, l'étape de personnalisation étant basée sur un ou plusieurs des éléments parmi (i) des paramètres cardiaques obtenus à partir d'un échocardiogramme, (ii) un signal ECG obtenu à partir d'un dispositif vestimentaire porté par le sujet lors de la mise en oeuvre de l'activité, et (iii) des métadonnées du sujet, y compris la taille et le poids qui lui sont associés.

SYSTEM **100**

MEMORY **102**

HARDWARE PROCESSOR(S) **104**

I/O INTERFACE(S) **106**

FIG.1

FIG.2

300

estimating, via one or more hardware processors, a plurality of input parameters for a personalized cardiovascular hemodynamic model associated with a subject being monitored (302), the plurality of input parameters comprising (i) Systemic Vascular Resistance (SVR), (ii) unstressed blood volume, (iii) total blood volume in a body of the subject, and (iv) heart rate of the subject

302

estimating, via the personalized cardiovascular hemodynamic model, cardiac parameters including cardiac output, ejection fraction and mean arterial pressure, using the estimated plurality of input parameters

304

estimating the SVR using Metabolic EquivalenT (MET) levels associated with an activity level of the subject

302a

updating the unstressed blood volume estimated when the subject is at rest, using an autoregulation method, when an activity is performed by the subject

302b

FIG.3A           A

```
                              ( A )
                                │
                                ▼
┌────────────────────────────────────────────────────────────────────────────┐      302b-1
│        sensing aortic pressure by baroreceptors located at carotid sinus and aortic arch        │
└────────────────────────────────────────────────────────────────────────────┘
                                │
                                ▼
┌────────────────────────────────────────────────────────────────────────────┐      302b-2
│     converting the sensed aortic pressure into a neural firing frequency via afferent sympathetic pathways     │
└────────────────────────────────────────────────────────────────────────────┘
                                │
                                ▼
┌────────────────────────────────────────────────────────────────────────────┐      302b-3
│   generating sympathetic and parasympathetic nervous activities via a central nervous system and efferent   │
│                     pathway depending on the neural firing frequency                     │
└────────────────────────────────────────────────────────────────────────────┘

┌────────────────────────────────────────────────────────────────────────────┐      302b-4
│  updating an additional blood demand representing the unstressed blood volume during the activity using the  │
│                generated sympathetic and parasympathetic nervous activities                │
└────────────────────────────────────────────────────────────────────────────┘
```

FIG.3B

FIG.4

FIG.5

```
┌─────────────┐      ┌─────────────┐
│             │      │ Estimation of│
│  MET Level  │─────▶│     SVR     │
│             │      │             │
└─────────────┘      └─────────────┘

┌─────────────┐                              ┌──────────────────┐      ┌─────────────┐
│  Heart rate │                              │   Personalized   │      │   Cardiac   │
│  estimation │─────────────────────────────▶│  Cardiovascular  │─────▶│  parameters │
│ from ECG/   │                              │   hemodynamic    │      │             │
│  PPG signal │                              │      model       │      └─────────────┘
└─────────────┘                              └──────────────────┘

┌─────────────┐      ┌─────────────┐           ┌─────────────┐
│  Height &   │      │ Total blood │           │  Unstressed │
│   Weight    │─────▶│   volume    │           │ blood volume│
│             │      │             │           │             │
└─────────────┘      └─────────────┘           └─────────────┘
```

FIG.6

FIG.7

FIG.8

FIG.9

FIG.10

FIG.11

FIG.12

FIG.13     Time (sec)

FIG.14

FIG.15

FIG.16

FIG.17

FIG.18

FIG.19

FIG.20

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- IN 202121010972

**Non-patent literature cited in the description**

- **ROY DIBYENDU et al.** Multimodal cardiovascular model for hemodynamic analysis: Simulation study on mitral valve disorders. *PLOS ONE,* 04 March 2021, vol. 16

- **ROY DIBYENDU et al.** Hemodynamic Evaluation of a Novel Neuromodulation based Adaptive controller for Mitral Stenosis. *2020 EUROPEAN CONTROL CONFERENCE (ECC), EUCA,* 12 May 2020